# EUROPEAN PATENT APPLICATION

(11) **EP 1 674 104 A1**
(43) Date of publication of application: **28.06.2006**
(21) Application number: 04356207.3
(22) Date of filing: 24.12.2004
(51) Int. Cl.: A61K 31/70, A61P 31/12

(54) **Uridine derivatives as antiviral drugs against a flaviviridae, especially HCV**

(71) Applicant: INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75016 Paris (FR)
(72) Inventor: Aucagne, Vincent, FR 28300 Amilly (FR); Escuret, Vanessa, FR 69003 (FR); Zoulim, Fabien, FR 69570 Dardilly (FR); Durantel, David, FR 69003 Lyon (FR); Trepo, Christian, FR 69500 Bron (FR); Agrofoglio, Luigi, FR 06600 Antibes (FR); Joubert, Nicolas, FR 45650 St Jean le Blanc (FR); Amblard, Franck, FR 36330 Le Poinconnet (FR)
(74) Representative: Colombet, Alain André

(57) **Abstract**

The invention relates to the use of an uridine derivative of formula (I): 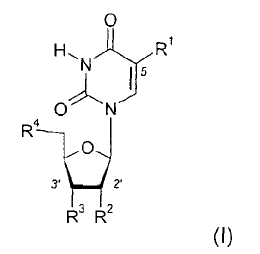 wherein
- R¹ represents monohalogenated alkynyl or dihalogenated alkenyl;
- R² is chosen from among hydrogen, hydroxyl, -O-alkyl, -O-CO-alkyl and halogen;
- R³ is chosen from among hydrogen, hydroxyl, -O-alkyl, -O-CO-alkyl, halogen, -SH, -S-alkyl and N³; and
- R⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms,
for the preparation of a drug having antiviral activity against a Flaviviridae.

## Description

The present invention relates to the use of uridine derivative(s) for the preparation of a drug having antiviral activity against a Flaviviridae, e.g. against hepatitis C virus (HCV). It also relates to new compounds having such antiviral activity, to drugs incorporating them and to a method of treating a subject (a human or an animal) with such a drug.

Hepatitis C Virus (HCV) infection remains a main public health problem with 175 million people infected in the world. Currently, the best treatment available consists in the association of pegylated interferon (IFN) alpha 2b and ribavirin (1-β-D-ribofuranosyl-1,2,4-triazole-3-carboxamide). 20% of patients infected with HCV genotype 2 or 3 and 50% of patients infected with HCV genotype 1 are non responders to this treatment. Moreover IFN and ribavirin are responsible for numerous adverse effects. Therefore new treatment regimens based on more potent and specific inhibitors of HCV replication are required to improve the current antiviral strategies against chronic HCV infection.

The absence of a cellular system able to allow a complete multiplication cycle of HCV *in vitro* is a limiting factor for the search of new drugs. Recently, major knowledge has been gained about the mechanism of HCV replication. Study models have been developed such as the replicon system described in Ju-Tao Guo et al. (J. Virol. 2001, 75, 18: 8516-8523), allowing replication of HCV subgenome in cell culture.

The recent development of the replicon system has promoted the study of new inhibitors of HCV replication. The NS3 serine protease and NTPase/helicase and the RNA-dependent RNA polymerase NS5B are key enzymes for the replication of HCV. To date, several inhibitors of NS3 were discovered. The NS5B has also been studied because of its central role in viral replication (Behrens, S.E. et al. *EMBO J*., **1996,** *15,* 12; Ferrari, E. et al. *J. Virol*., **1999,** 73, 1649; Ishii, K. et al. *Hepatology* **1999,** 29, 1227; Lohmann, V. et al. *J. Virol*., **1997,** 71, 8416; Oh, J.W. et al. *J. Virol*.,; **1999,** 73, 7694; Yamashita, T. et al. *J. Biol. Chem*., **1998,** 273, 15479; Walker, M.P.; Hong, Z., *Curr. Opinion Pharmacol.* 2002, 2, 1). Recently Bressanelli *et al*. reported a first co-crystallization of HCV NS5B with ribonucleotides, which lead to the identification of a specific and unique GTP-binding pocket outside the active site (Bressanelli, S. et al. J. *Virol*., **2002,** 76, 3482). Specific inhibitors of this virus-encoded RNA-dependent RNA polymerase (RdRP) may represent potent antiviral agents (De Francesco, R. et al. *Antiviral Res.* **2003,** 58, 1-16.

The pharmaceutical importance of nucleoside analogues against viral DNA polymerases such as HIV-RT, HBV and HSV polymerases, has prompted the design and synthesis of analogous viral RNA polymerase. While numerous of non-nucleoside inhibitors of NS5B-mediated HCV RNA replication have been reported (Chan, L. et al. J. *Med. Chem.* 2003, 46, 1283-1285; Wang, M. et al. J. *Biol. Chem.* **2003,** *278,* 9489-9495; Dhanak, D. et al. J. *Biol. Chem*., **2002,** *277,* 38322-38327), only a few nucleoside inhibitors have been identified so far (Carroll, S. S. et al J. *Biol. Chem.,* **2003,** *278*, 11979-11984; Shim, J. et al. *Antiviral Res.* **2003,** 243-251; Eldrup, A. B. et al. J. *Med. Chem*., **2004,** 47- 2283-2295). An example of nucleoside inhibitor is the 2'-C-methylarabinoguanosine (2'-C-methylaraG), recently reported in WO 01/190121, which is phosphorylated in cultured, uninfected cells and orally bioavailable in primates.

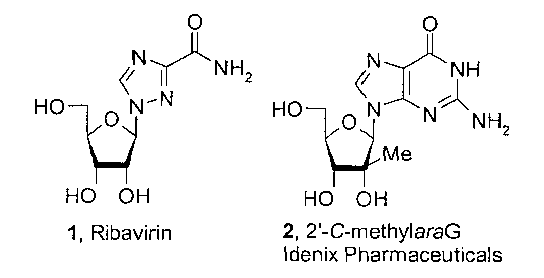

Many nucleoside analogues substituted at the 5-position of the heterocycle, and especially in the 2'-deoxyuridine series (e.g. BVDU, IDU, FIAU), are known to have potent biological properties and have been developed as antiviral and anticancer agents. The 5-(2-substitutedvinyl)-2'-deoxyuridines have emerged as potent and selective inhibitors of herpes viruses (HSV-1 & HSV-2) (De Clercq, E., *Exp. Clin. Pharmacol.* **1980**, 2, 253; De Clercq, E., *Biochem. Pharmacol.* **1984**, 33, 2159). In general, 5-alkynyl-2'-deoxyuridines has been also evaluated as potential antiviral agents (De Clercq, E. et al *Proc. Natl. Acad. Sci. U.S.A.* **1979,** 76, 2947; De Clercq, E.et al. *J*. *Infect. Dis*., **1980,** *141*, 563; Kundu, N. G.; Dasgupta, S. K., J. *Chem. Soc., Perkin Trans.* 1, **1993**, 2657). The structure activity relationship studies seem to indicate that the type of C-5 substituents likely to confer activity are those which are electron withdrawing and conjugated to the heterocycle (Goodchild, J. et al. *J. Med. Chem*. **1983,** *26,* 1252). Recently, McGuigan *et al*. reported that analogous furanopyridine by-product in the preparation of the parent 5-alkynyl-2'-deoxyuridine displayed important potency and exclusive selectivity for varicella zooster virus (VZV) (McGuigan C. et al. J. *Med. Chem.,* **1999,** 42, 4479; McGuigan, C. et al. J. *Med. Chem*. **2000,** *43*, 4993; Srinivasan, S. et al. *Bioorg. Med. Chem. Lett*. **2001,** *11*, 391; McGuigan, C.et al. *Drugs Future,* **2000**, *25*, 1151; Carangio, A. et al. *Antivir. Chem. Chemother.* **2001**, *12*, 187), meanwhile imidazo[1,2-c]pyrimidin-5(6H)-one heterosubstituted nucleoside analogues were discovered by Mansour *et al*. (*Bioorg. Med. Chem. Lett*. **1997,** *7*, 303) to have anti-HBV (hepatitis B virus) activity. The chemistry involves the Pd-catalyzed coupling of arylacetylenes or terminal alkynes with 5-iodonucleosides.

Providing the art with new efficient and acceptable anti-Flaviridae, in particular anti-HCV, drugs or treatments would be of great benefit.

An objective of the invention is to provide the art with new treatments effective against a Flaviviridae infection, e.g. against HCV, say new treatments having a strong antiviral activity with no or acceptable side effects.

Another objective of the invention is to provide a treatment that is efficient against the major HCV genotypes, including genotypes 1, 2 and 3.

Another objective of the invention is to provide a treatment that is efficient against any HCV genotype.

These objectives are attained by the use of some uridine derivatives that will be described in details below.

A first object of the invention is the use of a uridine derivative of formula (I): 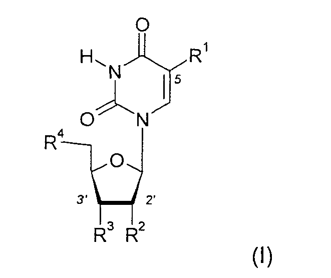 wherein
- R¹ represents monohalogenated alkynyl or dihalogenated alkenyl;
- R² is chosen from among hydrogen, hydroxyl, -O-alkyl, -O-CO-alkyl and halogen;
- R³ is chosen from among hydrogen, hydroxyl, -O-alkyl, -O-CO-alkyl, halogen, -SH, -S-alkyl and N³; and
- R⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms,
for the preparation of a drug having antiviral activity against a Flaviviridae.

In the above formula (I):
"monohalogenated alkynyl" is -C ≡C-X or -C ≡C-Ak(X), where Ak(X) denotes a linear or branched C₁-C₁₀ alkyl radical, one hydrogen atom of which being substituted by one halogen atom X;
"dihalogenated alkenyl" is -C(Y)=CH(X), -C(Y)=C(X)alkyl or -C(Y)=C-Ak(X), where Y is halogen and Ak(X) denotes a linear or branched C₁-C₁₀ alkyl radical, one hydrogen atom of which being substituted by one halogen atom X;
"alkyl", unless otherwise indicated, represents a linear or branched C₁-C₁₀ alkyl radical;
"halogen" is fluorine, chlorine, bromine or iodine, preferably chlorine, bromine or iodine.

The dihalogenated alkenyl may present a *Z* or *E* configuration, the *E* configuration being preferred, i.e. the two halogen atoms X and Y are not on the same side of the planar double bond.

Preferred configuration for -C(Y)=CH(X) or -C(Y)=C(X)alkyl is therefore respectively:

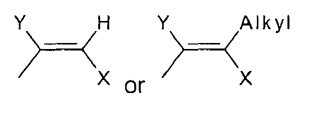

According to a first embodiment, a preferred uridine derivative of formula (I) useful for the preparation of a drug having antiviral activity against a Flaviviridae, presents the following characteristics:
- R¹ represents -C ≡C-X or -C(Y)=CH(X);
- X and Y each independently represents halogen;
- R² is chosen from among hydrogen, hydroxyl, -O-alkyl, -O-CO-alkyl and halogen;
- R³ is chosen from among hydrogen, hydroxyl, -O-alkyl, -O-CO-alkyl, halogen, -SH, -S-alkyl and N³; and
- R⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms.

Preferred compounds of formula (I) are those where
- R¹ represents chloroethynyl, bromoethynyl, iodoethynyl or -C(Y)=CH(X), where Y is chosen from among chlorine, bromine and iodine, and X is bromine or iodine,
- R², R³ and R⁴ being as defined above,
as well as their possible tautomers, their possible pharmaceutically acceptable addition salts with an acid or a base, and their N-oxide forms.

Compounds where R² and R³ do not simultaneously represent hydrogen form another subgroup of even more preferred compounds.

Particularly preferred compounds for the intended above-mentioned use are those wherein
- R¹ represents chloroethynyl, bromoethynyl, iodoethynyl or -C(Y)=CH(X), where Y is chosen from among chlorine, bromine and iodine, and X is bromine or iodine;
- R² is chosen from among hydrogen, hydroxyl, and -O-alkyl;
- R³ is chosen from among hydrogen, hydroxyl, -O-alkyl, -SH, and -S-alkyl;
- R² and R³ not simultaneously being each hydrogen; and
- R⁴ is chosen from among hydroxyl, -O-alkyl, -O-CO-alkyl, -O-phosphate,
- O-diphosphate, -O-triphosphate and -O-phosphonate, as well as their possible tautomers, their possible pharmaceutically acceptable additions salts with an acid or a base, and their N-oxide forms.

A most preferred uridine derivative of formula (I) is chosen from among the following compounds:
- (*E*)-5-(1-Chloro-2-iodovinyl)-2'-deoxyuridine,
- (*E*)-5-(1,2-Diiodovinyl)-2'-deoxyuridine,
- 5-(2-lodoethynyl)-2'-deoxyuridine,
- (*E*)-5-(1-Bromo-2-iodovinyl)-2'-deoxyuridine,
- (E)-5-(1,2-Dibromovinyl)-2'-deoxyuridine,
- 5-(2-Bromoethynyl)-2'-deoxyuridine,
- (*E*)-5-(1-Chloro-2-iodovinyl)-uridine,
- (*E*)-5-(1,2-Diiodovinyl)-uridine,
- 5-(2-lodoethynyl)-uridine,
- (*E*)-5-(1-Bromo-2-iodovinyl)-uridine,
- (*E*)-5-(1,2-Dibromovinyl)-uridine, and
- 5-(2-bromoethynyl)-uridine,
as well as its possible tautomers, its possible pharmaceutically acceptable addition salts with an acid or a base, and its N-oxide forms.

Compounds of formula (I) wherein R⁴ represents -O-phosphate, -O-diphosphate, -O-triphosphate and -O-phosphonate are deemed to represent pro-drugs of the corresponding compounds wherein R⁴ represents -OH. Other pro-drugs leading to compounds wherein R⁴ is -OH are also encompassed within the present invention. Such other pro-drugs include for example esters, such as aminoacid esters, e.g. alanine esters and glycine esters, quaternary salts of phosphate, and the like.

Acids that may be used to form pharmaceutically acceptable addition salts of the compound of formula (I) are organic or inorganic acids. Such salts include for example hydrochlorides, hydrobromides, sulfates, hydrogensulfates, dihydrogene-phosphates, citrates, maleates, fumarates, trifluoroacetates, 2-naphthalenesulfonate and para-toluenesulfonate.

Similarly, bases that may be used to form pharmaceutically acceptable addition salts of the compound of formula (I) are organic or inorganic bases. Such salts include for example metallic salts from alkali metals, alkaline-earth metals or transition metals, such as sodium, potassium, calcium, magnesium, aluminum. Other suitable bases include ammoniac or secondary or tertiary amines (diethylamine, triethylamine, piperidine, piperazine, morpholine) or basic amino-acids, or alternatively osamines (such as meglumine), or aminoalcohols (such as 3-aminobutanol and 2-aminoéthanol).

The present invention also encompasses salts that are useful for suitable separation or crystallization of the compounds of formula (I), such as salts obtained from chiral amines or acids.

Example of chiral amines include for example quinine, brucine, (S)-1-(benzyloxymethyl)propylamine, (-)-ephedrine, (4*S*,5*R*)-(+)-1,2,2,3,4-tetramethyl-5-phenyl-1,3-oxazolidine, (*R*)-1-phenyl-2-*p*-tolylethylamine, (*S*)-phenylglycinol, (-)-N-methylephedrine, (+)-(2*S*,3*R*)-4-dimethylamino-3-methyl-1,2-diphenyl-2-butanol, (*S*)-phenylglycinol, (*S*)-α-methylbenzylamine, alone or in mixtures.

Example of chiral acids include for example (+)-d-di-O-benzoyltartaric acid, (-)-I-di-O-benzoyltartaric acid, (-)-di-O,O'-p-toluyl-I-tartaric acid, (+)-di-O,O'-p-toluyl-d-tartaric acid, (R)-(+)-malic acid, (S)-(-)-malic acid, (+)-camphanic acid, (-)-camphanic acid, (+)-camphoric acid, (-)-camphoric acid, (*S*)-(+)-2-phenylpropionic acid, (*R*)-(-)-2-phenylpropionic acid, d-(-)-mandelic acid, I-(+)-mandelic acid, d-tartaric acid, I-tartaric acid, alone or mixtures.

In an embodiment, the invention more specifically relates to the use of such an uridine derivative and an efficient amount of an interferon (IFN), for the preparation of a drug having antiviral activity against a Flaviviridae. It also relates to the use of such an uridine derivative for the preparation of a drug having antiviral activity against a Flaviviridae, that is intended to be used in a recipient that is treated with an efficient amount of IFN. The recipient may have been treated recently with the IFN (for instance less than 30 days before), or he receives IFN at approximately the same time or later, for instance less than 30 days. The IFN may be chosen among those that are described *infra.*

In another embodiment, the IFN is replaced or accompanied (concomitant or successive administration) by the use of another drug such as one of those described *infra.*

According to a preferred aspect, the Flaviridae is HCV and the use of a uridine derivative of formula (I) is for the preparation of a drug having antiviral activity against HCV. There exists up to 6 HCV genotypes classified 1-6. Any of them is included in the present invention. The major genotypes are genotypes 1, 2 and 3, and a "use" according to the invention may be, for example, directed against HCV genotype 1, HCV genotype 2, HCV genotype 3, HCV genotypes 1 and 2, HCV genotypes 2 and 3, HCV genotypes 1 and 3, HCV genotypes 1, 2 and 3. The use may also be against any and all HCV genotypes, including quasispecies.

In another aspect of the invention, the Flaviviridae member may be a member of the Japanese encephalitis virus group, including Japanese encephalitis virus and West Nile Virus.

Alternatively it may be a member of the Yellow fever virus group.

Alternatively it may be a member of the Pestivirus group, such as Bovine viral diarrhea virus (BVDV-1 and/or BVDV-2), Classical swine fever virus, Border disease virus.

It may be appreciated that the recipient may be a human or an animal, depending on the Flaviviridae concerned.

Among the compounds of formula (I), 5-bromoethynyl-2'-deoxyuridine is known and reported by Eger K. *et al*. (*Tetrahedron,* **1994,** *50,* 8371-8380), whereas compounds of formula (I), wherein R¹ represents dihalogenated alkenyl are new.

Therefore, a further object of the present invention is a compound of formula (I'): 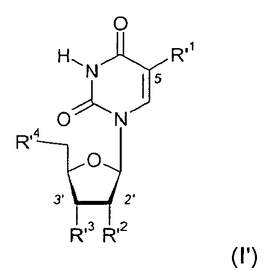 wherein
- R'¹ represents dihalogenated alkenyl;
- R'², R'³ and R'⁴ have the same definitions as R², R³ and R⁴, respectively, in the above formula (I);
as well as its possible tautomers, its possible pharmaceutically acceptable addition salts with an acid or a base, and its N-oxide forms.

In the above formula (I'), "dihalogenated alkenyl" has the same meaning as defined above, i.e. -C(Y)=CH(X), -C(Y)=C(X)alkyl or -C(Y)=C-Ak(X), where Y is halogen and Ak(X) denotes a linear or branched C₁-C₁₀ alkyl radical, one hydrogen atom of which being substituted by one halogen atom X;

All definitions and preferred embodiments described hereinbefore for compounds of formula (I) also apply to compounds of formula (I'), R'¹ not possibly representing however a monohalogenated alkynyl radical.

Accordingly, a preferred compound of formula (I') presents the following characteristics:
- R'¹ represents -C(Y)=CH(X);
- X and Y each independently represents halogen;
- R'² is chosen from among hydrogen, hydroxyl, -O-alkyl, -O-CO-alkyl and halogen;
- R'³ is chosen from among hydrogen, hydroxyl, -O-alkyl, -O-CO-alkyl, halogen, -SH, -S-alkyl and N³; and
- R'⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms.

Preferred compounds of formula (I') are those where:
- R'¹ represents -C(Y)=CH(X), where Y is chosen from among chlorine, bromine and iodine, and X is bromine or iodine.
- R'², R'³ and R'⁴ being as defined above,
as well as their possible tautomers, their possible pharmaceutically acceptable addition salts with an acid or a base, and their N-oxide forms.

Particularly preferred compounds for the intended above-mentioned use are those wherein
- R'¹ represents -C(Y)=CH(X), where Y is chosen from among chlorine, bromine and iodine, and X is bromine or iodine.
- R'² is chosen from among hydrogen, hydroxyl, and -O-alkyl;
- R'³ is chosen from among hydrogen, hydroxyl, -O-alkyl, -SH, and -S-alkyl; and
- R'² and R'³ not simultaneously being each hydrogen; and
- R'⁴ is chosen from among hydroxyl, -O-alkyl, -O-CO-alkyl, -O-phosphate, -O-diphosphate, -O-triphosphate and -O-phosphonate,
as well as their possible tautomers, their possible pharmaceutically acceptable addition salts with an acid or a base, and their N-oxide forms.

The present invention is further related to the process for the preparation of compounds of formula (I) and formula (I').

Halogenated pyrimidine **4a-c** in Scheme 1 below are prepared by direct reaction with halogens following the procedure reported by Asakura et al. (*J. Org. Chem*., **1990,** *55*, 4928-4933) *via* a cerium(IV)-mediated halogenation at the C-5 of uracil derivatives. Thus, treatment of the known acetylated nucleosides **3a-c** according to a process well known by the one skilled in the art, with elemental iodine and ceric ammonium nitrate (CAN) at 80°C, gives the corresponding protected 5-iodouracil nucleosides 4a-c in excellent yields. This CAN-mediated halogenation has advantages over other available methods as mild reaction conditions are employed with short reaction times.

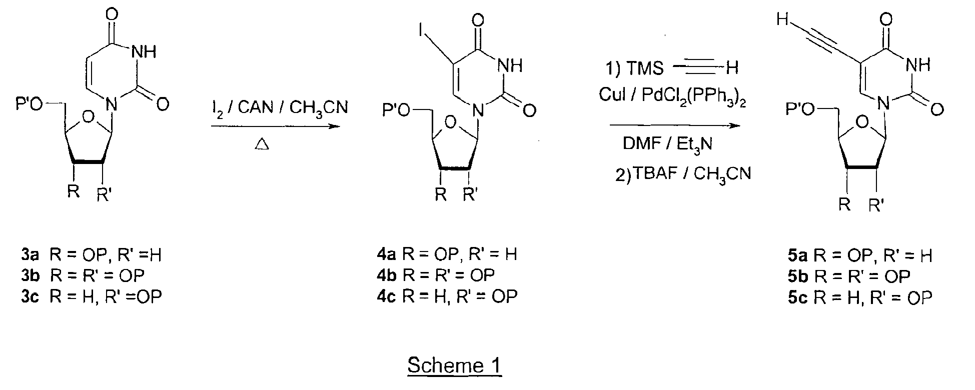

In the above scheme 1, P and P', which may be identical or different, stand for protective groups of hydroxyl groups, and are well-known to the person skilled in the art.
Preferably, protective groups P and P' are chosen from among acyl groups such as CH₃C=O, trityl, monomethoxytrityl, mesyl, and the like.
In scheme 1 above, protective groups P and P' each preferably an acyl group, specifically CH₃C=O.

The introduction of a C-5-alkynyl group is performed by a Pd(0)-mediated reaction, using the *Sonogashira conditions* (Sonogashira K., *Comprehensive Organic Synthesis,* Trost/Fleming (Eds), Pergamon Press, New York, **1991,** vol 3, p. 521;. Sonogashira K. et al.; *Tetrahedron Lett*., **1975,** 16, 4467; Takahashi S. et al., *Synthesis*, **1980,** 627; Ratovelomana V. et al., *Synth. Commun*., **1981,** *11*, 917). Thus, reaction of the resulting pure acetylated 5-iodouracil **4a-c** with trimethylsilylacetylene in the presence of triethylamine (Et₃N, base), cuprous iodide (Cul, co-catalyst) and PdCl₂(PPh₃)₂ (catalyst) in anhydrous dimethyl formamide (DMF) at room temperature, followed by a removal of the trimethylsilyl (TMS) group with TBAF (tetrabutyl ammonium fluoride) affords the corresponding 5-ethynyluracil nucleosides **5a-c**. In most cases, it is observed that slight elevations in the reaction temperature lead to increase in the rate of coupling. In all cases, a by-product, furanopyrimidine derivatives, is either isolated with a < 7% yield, or just detected by TLC; nevertheless, the using of DMF reduces the amount of this cyclic by-product; this base-catalyzed cyclization was previously reported by Bleackley *et al*. *(Tetrahedron,* **1976,** *32,* 2795) by base treatment of (E)-5-(2-bromovinyl)uracil.

The halogenation of alkynes **5a-c** is realized using halogenating agents (such as iodonium di-syn-collidine perchlorate (IDCP), N-iodosuccinimide (NIS) or chloroiodine (ICL) for iodination, bromonium di-syn-collidine perchlorate (Br(coll)₂ClO₄) for bromination, or, for chlorination, sulfuryl chloride, Cl₂, CCl₄, N-chlorophtalimide, dichlorine monoxide or PCl₅, in anhydrous polar solvent, for example methane nitrile (CH₃CN) according to reaction Scheme 2, where P and P' are as defined above for Scheme 1. These halogenating reagents are known to act as very reactive electrophiles (such as I⁺ and Br⁺, respectively).

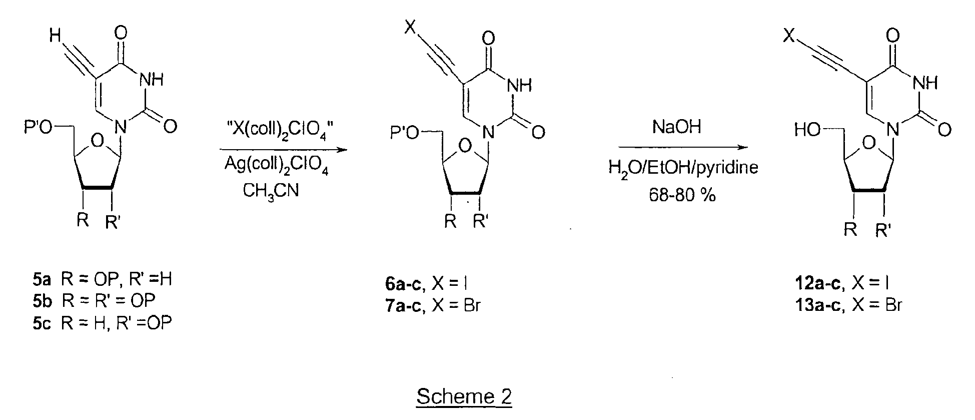

The final deprotection of protected nucleosides **6a-c** and **7a-c** is realized under smooth basic conditions (for example 1M sodium hydroxide in a mixture of H₂O/EtOH/pyridine when P = P' = Ac) to afford final 5-haloethynyluracil nucleosides **12a-c** and **13a-c**, respectively, in good to moderate yield.

Dihalogenation of the above 5-ethynyl nucleosides (Scheme 3) leads to hitherto unknown 5-(1,2-dihalogenated)ethenyluracil nucleosides **14a-c** to **17a-c.** Several halogenating reagents (i.e. I₂, IBr, ICI or Br₂) are used to reach the corresponding 1,2-dihaloderivatives. In all cases of halo-iodination, the formation of vicinal halo-iodoalkenes (**8a-c to 10a-c**) occurs with high anti-stereospecificity, implicating the intermediary of an iodonium ion in the reaction sequence, and a high and unique regioselectivity, implicating a predominantly Markovnikov addition. The formation of **11a-c** using Br₂ occurs also with a *E*-stereochemistry.

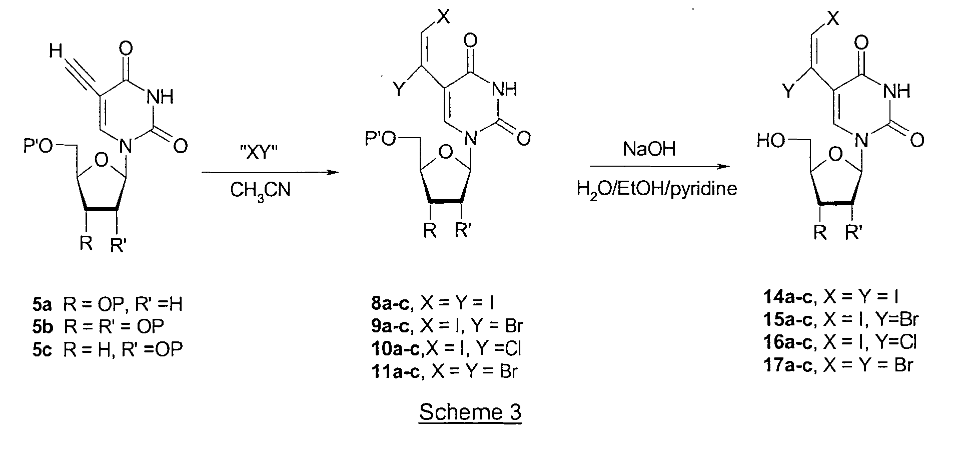

The final deprotection of protected nucleosides **8a-c** to **11a-c** is realized as previously reported (for example 1M NaOH in a mixture of H₂O/EtOH/pyridine when P=P'=Ac) and final 5-(1,2-dihaloethenyl)uracil nucleosides **14a-c** (for X = Y = I), **15a-c** (for X = I, Y = Br), 16a-c (for X = I, Y = Cl), **17a-c** (for X = Y = Br) are obtained, respectively.

All other compounds encompassed by formulae (I) and (I') are easily prepared from the above described synthetic route with easily conceivable modifications and/or adaptations well known by the one skilled in the art.
For illustrative purposes only, compounds of formula (I) or of formula (I') may be obtained according to scheme 4 below, wherein P preferably represents mesyl and P' preferably represents monomethoxytrityl.

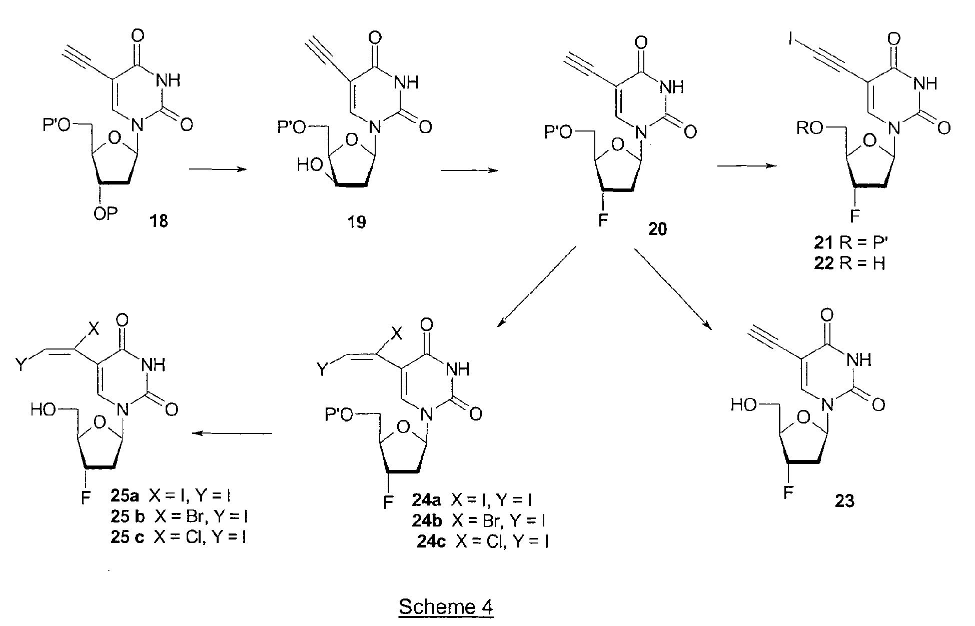

In scheme 4 above, compound **19** is obtained after specific deprotection, in mild basic conditions, the free resulting hydroxyl group being substituted by methods well known in the art, for example using diethylaminosulfur trifluoride, affording compound **21.**

Compounds **21** and **22** are obtained from compound **20** under conditions similar as those described above for the preparation of compounds **12** and **13.**

Compound **23** results from the deprotection of the P' protecting group of compound 20.

Compounds **24a-c** and **25a-c** are respectively obtained using a similar process as the one disclosed above for the preparation of compounds **8-11a**-**c** and **14-17a-c**.
As further illustrative purpose only, compounds of formula (I) and of formula (I') wherein R³ (and R¹³ respectively) represents N₃ may be obtained according the following scheme 5.

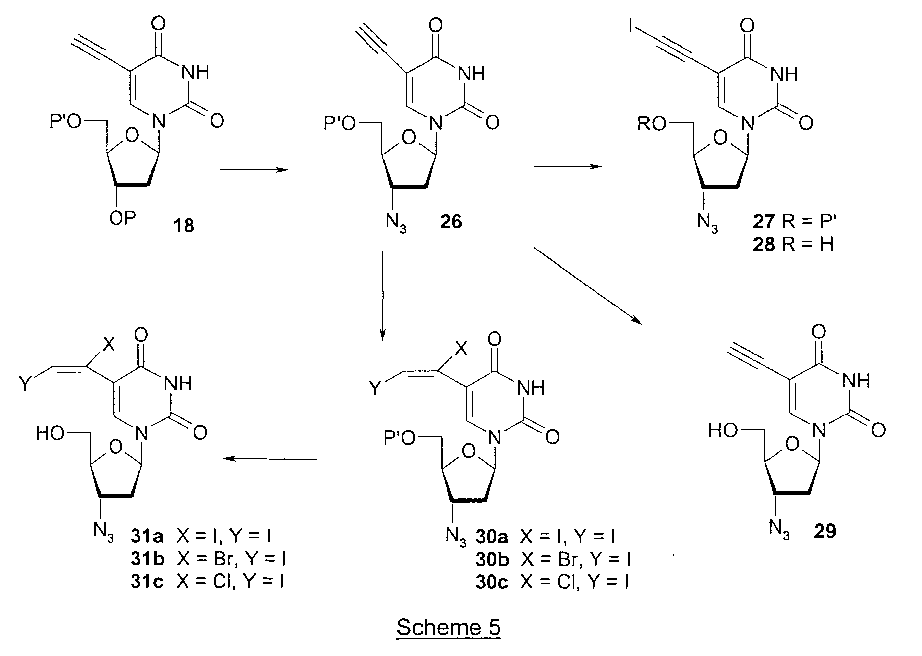

In the above scheme 5, P and P' each denote protective groups as defined above and advantageously P represents mesyl and P' represents monomethoxytrityl.

Azidation of compound **18** may be carried out using conventional techniques of azidation and for example using sodium azide in polar aprotic solvent, such as for example dimethylformamide under reflux, resulting in compound 26.

Compounds **27-31** are obtained through similar methods as those disclosed above for the preparation of compounds **21-25** respectively.

Compound **18** in scheme 4 and scheme 5 above may be obtained according to a process similar to the process for the preparation of compound 5 in the above scheme 1.

Alternatively, compound of formula 18 may advantageously be obtained according to the preparation process of scheme 6 below:

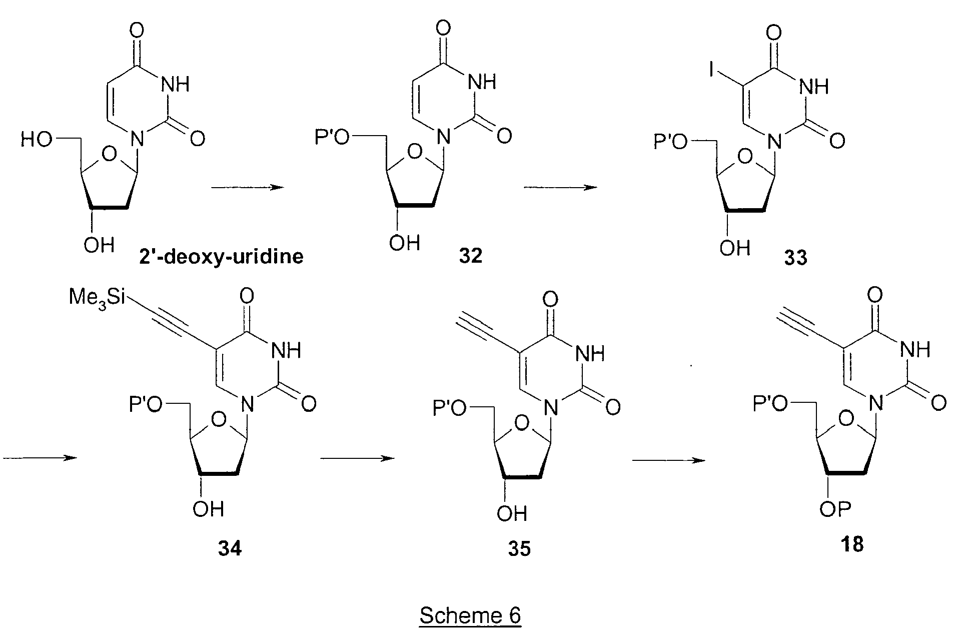

In scheme 6 above, P and P' are preferably different, each representing a protective group as defined above. Particularly preferred are compounds wherein P' represents monomethoxytrityl and P represents mesyl.

The introduction of the P' protective group is carried out using conventional techniques, and for example, where P' represents monomethoxytrityl using para-anisylchlorodiphenylmethane in mild basic conditions, such as dry pyridine at reflux.

The selective introduction of protective group P is also achieved using conventional techniques, and, for example, when P represents mesyl, using methanelsulfonylchloride in mild basic conditions, such as for example dry pyridine at 0 °C.
In scheme **6** above, the introduction of a C-5-alkynyl group is performed as stated above in scheme 1 for the preparation of compound **5.**

Compounds **14-31 a-c** are purified, when necessary, using conventional methods known in the art.

Compounds of formulae (I) and (I') may be obtained in pure enantiomeric form and/or isomeric form, or in mixture, in all proportions, of enantiomers and/or isomers. Separations of such enantiomers and/or isomers is run according to techniques well known in the art.

The above compounds are also optionally converted into pharmaceutically acceptable addition salts with an acid or a base, and optionally into their N-oxide form, using appropriate and suitable methods as known by the one skilled in the art.

All starting materials are known and either commercially available or prepared, directly or with slight variations easily conceivable by the one skilled in the art, from one or more processes disclosed in the scientific literature, patents and patent applications, the "Chemical Abstracts", on-line data bases and the Internet.

Another object of the invention is a drug or drug formulation having an antiviral activity against a Flaviviridae, in particular HCV, comprising at least one compound of formula (I) (the active ingredient) with a pharmaceutically acceptable vehicle, carrier or excipient. "Acceptable" means that this further ingredient is compatible with the active ingredient included in the formulation and is not deleterious to the recipient thereof. The pharmaceutically acceptable vehicle, carrier or excipient are well known to the one skilled in the art. For example, for parenteral administration, it can be a 0.9% NaCl saline solution or a phosphate buffer. The pharmaceutically acceptable vehicles, carriers or excipients also cover any compound or combination of compounds facilitating the administration of the active ingredient and/or improving preservation.

It is noted that in this disclosure and particularly in the claims, terms such as "comprises", "comprised", "comprising" and the like can have the meaning attributed to it in U.S. Patent law; e.g., they can mean "includes", "included", "including", and the like.

The formulations include those suitable for oral, rectal, nasal, topical (including buccal or sublingual), vaginal or parenteral (including subcutaneous, intramuscular, intravenous, intradermal, intrathecal and epidural) administration. These various formulations are well known, as well as their methods of preparation. The choice of the formulation may vary depending upon the identity of the recipient, and will generally be chosen according to the best practice for a human or for the animal considered. The formulations may conveniently be presented in unit dosage form and may be prepared by any of the methods well known in the art of pharmacy. Formulations for oral administration may be presented as discrete units such as, for example: capsules, cachets or tablets each containing a predetermined amount of active ingredient; a powder or granules; a solution or a suspension in an aqueous liquid or a non-aqueous liquid; or an emulsion, e.g. a water-in-oil or an oil-in-water emulsion. The active ingredient may also be presented as a bolus, electuary or paste. The solid forms may optionally be coated and may be formulated so as to provide slow or controlled release of the active ingredient. For infections of the eye or other external tissues (e.g. skin or mouth), the formulations are preferably presented as ointment or cream for topical application, containing an efficient amount of active ingredient. Formulations suitable for administration to the eye also include eye drops where the active ingredient is dissolved or suspended in a suitable carrier, such as an aqueous solvent. Formulations for rectal administration may be presented as a suppository with a suitable base. Liquid or solid carriers may be used for nasal administration. Pessaries, tampons, creams, gels, pastes, foams or spray formulations may be used for vaginal administration. Formulations for parenteral administration include aqueous and non-aqueous sterile injection solutions, suspensions or emulsions.

The amount of active ingredient in a single dosage form will depend upon a number of factors including the identity of the recipient and the condition to be treated. In general, however, for a human, the amount of active ingredient in a single dose form will be in the range, for example, of 1 mg to 10 g, preferably in the range of 100 mg to 5 g, most preferably in the range of 500 to 2000 mg.

For multiple dosage form, for example a cream or ointment, the concentration of active ingredient will depend upon a number of factors including the identity of the recipient and the condition to be treated. In general, however, for a human, the concentration of active ingredient in a multiple dose form will be in the range, for example, of 0.01 to 200 mg of active ingredient per g of formulation, preferably in the range of 0.1 to 100 mg/g, most preferably in the range of 1 to 50 mg/g.

In one embodiment, the drug formulation according to the invention further comprises an additional compound having an antiviral activity against a Flaviviridae or that is useful in the treatment of the infection or its symptoms. The use according to the invention thus encompasses the use of at least two different active ingredients, at least one of them being an uridine derivative as described herein.

In a first aspect, this additional compound is at least one formula (I) compound. Thus the invention formulation does comprise at least two formula (I) compounds or derivatives.

In a second aspect, the drug formulation having an antiviral activity against a Flaviviridae comprises an, additional compound which is not an uridine derivative according to the invention. The drug formulation may be then represented as comprising:
(1) an uridine derivative of formula (I): 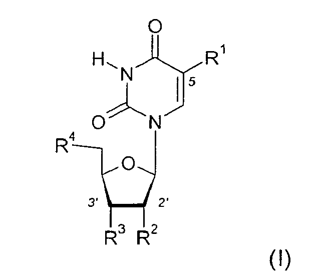 wherein
   - R¹ represents monohalogenated alkynyl or dihalogenated alkenyl;
   - R² is chosen from among hydrogen, hydroxyl, -O-alkyl, -O-CO-alkyl and halogen;
   - R³ is chosen from among hydrogen, hydroxyl, -O-alkyl, -O-CO-alkyl, halogen, -SH, -S-alkyl and N³; and
   - R⁴ is chosen from among hydroxyl, -O-alkyl, -O-CO-alkyl, -O-phosphate, -O-diphosphate, -O-triphosphate and -O-phosphonate,
   as well as its possible tautomers, its possible pharmaceutically acceptable addition salts with an acid or a base, and its N-oxide forms,
(2) an immunomodulator or another active ingredient having an antiviral activity against a Flaviviridae or being useful in treating the infection by a Flaviridae, and
(3) a pharmaceutically acceptable vehicle, carrier or excipient.

The additional compound is advantageously an immunomodulator, especially an interferon (IFN) or a derivative of an interferon such as the so-called pegylated forms of interferon (pegylated interferon), such as any of those currently used in the medical field. It may be in particular IFN-*α*, e.g. IFN-*α* 2b, such as pegylated IFN-*α* 2b Peg-Intron® (Schering Plough), IFN-*α* 2a, such as pegylated IFN-*α* 2a Pegasis® (Roche), Albuferon-a® (Human Genome Sciences), Multiferon® (Viragen); IFN-*β*, e.g. IFN β 1a, such as Rebif® IFN-*β*1a (Serono); IFN-*γ*, e.g. IFN-*γ* 1b, such as IFN-*γ* 1b *(*from Interimmune Pharmaceuticals; IFN-*ω*, e.g IFN-*ω* from Biomedines.

Other immunomodulators may be associated to the invention compounds, such as histamine dihydroch.loride (e.g. Ceplene®, Maxim Pharmaceuticals), Thymosine-a (SciClone Pharmaceuticals), Interleukines such as IL-12 and IL-10, therapeutical vaccines (e.g. based on the E1 protein), preparation for passive immunity transfer (e.g. Cicavir®, NABI).

More generally, the invention compounds may find an interest in the association to any available drug used in the treatment of the infection by a Flaviridae, especially HCV. Thus, the drug formulation may include in addition an active principle chosen among antisens molecules, protease inhibitors, helicase inhibitors, polymerase inhibitors, nucleoside or nucleotide analogs (chain terminators, ribonucleoside analogs, sugar modified nucleoside analogs), ribavirine or ribavirine pro-drug, inosine monophosphate dehydrogenase inhibitors.

The invention encompasses of course combinations of at least two different additional compounds with an uridine derivative according to the invention.

In a third aspect, the drug or drug formulation according to the invention comprises at least two formula (I) compounds and at least one additional compound as described above, in particular an IFN or derivative thereof.

Another object of the invention is thus a drug formulation comprising at least one formula (I) compound, at least one additional compound as described *supra,* in particular an IFN or derivative thereof, and a pharmaceutically acceptable vehicle, carrier or excipient, as described *supra.*

Another object of the invention is a method for antiviral treatment of a recipient (a human or an animal), wherein the recipient is administered with an efficient amount of a drug or drug formulation according to the invention.

In an embodiment, the method includes the administration of an efficient amount of uridine derivative(s) and an efficient amount of interferon(s) (IFN). The uridine derivative and the IFN may be in admixture, or may be separately formulated and are either co-injected after extemporaneous mixture, or administered separately to the recipient. As an alternative, the method provides for the administration of an efficient amount of uridine derivative(s), where this administration is intended to be made in a recipient that is treated with an efficient amount of IFN(s). The recipient may have been treated recently with the IFN(s) (for instance less than 30 days before), or he receives IFN(s) at approximately the same time or later, for instance less than 30 days. In another embodiment, the IFN is replaced or accompanied (concomitant or successive administration) by the administration of another drug such as one of those described *supra.* Any and all characteristics recited with respect to the use according to the invention apply to the method.

As mentioned above, the compounds or derivatives, and the formulations or compositions comprising them may be administered by any route appropriate to the condition to be treated. Suitable routes include oral, rectal, nasal, topical (including buccal or sublingual), vaginal or parenteral (including subcutaneous, intramuscular, intravenous, intradermal, intrathecal and epidural). It will be appreciated that the preferred route may vary with, for example, the condition of the recipient. Various suitable formulation types have been described earlier and may be used in the method or use.

For each of the above-indicated infectious events or indications the amount required of the active ingredient will depend upon a number of factors including the severity of the condition to be treated and the identity of the recipient and will ultimately be at the discretion of the attendant physician. In general, however, for each of these indications, a suitable, effective dose will be in the range 0.01 to 200 mg per kilogram body weight of recipient per day, preferably in the range of 2 to 100 mg, most preferably 10 to 40 mg per kilogram body weight of recipient per day. The dose may if desired be presented as two, three, four or more sub-doses administered at appropriate intervals throughout the day.

The invention is described further in details with the following description of non-limitative examples.

### EXAMPLES

Commercially available chemicals and solvents are reagent grade and used as received. Dry tetrahydrofuran, pyridine and dichloromethane are obtained from distillation over calcium hydride (CaH₂) or sodium (Na), *N,N*-dimethylformamide and ethanol over barium oxide (BaO) or over calcium hydride (CaH₂). Triethylamine is dried over potassium hydroxide (KOH). The reactions are monitored by thin-layer chromatography (TLC) analysis using silica gel plates (Kieselgel 60 F₂₅₄, E. Merck). Compounds are visualized by UV irradiation and/or spraying with 20% sulphuric acid (H₂SO₄) in ethanol (EtOH), followed by charring at 150 °C. Column chromatography is performed on Silica Gel 60 M (0.040-0.063 mm, E. Merck). Melting points (mp) are recorded on a Büchi (Dr. Tottoli) and are uncorrected. ¹H and ¹³C NMR spectra are recorded on a Bruker AVANCE DPX 250 Fourier Transform spectrometer at 250 MHz for ¹H and 62.9 MHz for ¹³C respectively, in (D)-chloroform, (D₄)-methanol and (D₆)-DMSO, shift values in ppm relative to SiMe₄ as internal reference, unless otherwise stated ; signals are reported as s (singlet), d (doublet), t (triplet), q (quartet), m (multiplet), *J* in Hz. [α]_{D} were performed with a Perkin-Elmer (model 41) polarimeter. UV spectra were recorded on a Beckman DU-640 spectrophotometer. Mass spectra are recorded on a Perkin-Elmer SCIEX API-300 (heated nebullizer) spectrometer. High Resolution Mass spectra (HRMS) were performed by the Centre Regional de Mesures Physiques de l'Ouest, University of Rennes 1 (France) using the FAB (Fast Atom Bombardment) or ESI (Electron Spray Ionization) mode. The nomenclature of the obtained compounds is in accordance with the IUPAC rules. The numbering and assignment of the chemical shifts for all described compounds are related to the corresponding ribose derivatives.

### Preparation of compounds of formula (I)

### General procedure for iodination of acetylated nucleosides

A solution of acetylated nucleoside **3a-c** (synthetic route described by L. B. Townsend and R. S. Tipson in "Nucleic acid chemistry", Wiley Eds., 1978) (15 mmol) in dry methane nitrile (CH₃CN, 150 mL), ceric ammonium nitrate (CAN, 4.94 g, 0.6 eq.) and iodine (I₂, 2.28 g; 0.6 eq.) is refluxed until completion (typically 1 h, checked by TLC). After cooling to room temperature (rt), solvents are evaporated under reduced pressure and the dark oily residue is dissolved in ethyl acetate (AcOEt, 300 mL) and water (H₂O, 50 mL). The biphasic mixture is cooled in an ice bath and a saturated Na₂S₂O₃ solution is smoothly added until complete decolouration. The organic layer is washed with water (2 x 50 mL) and brine (50 mL), dried over magnesium sulfate (MgSO₄) and concentrated under reduced pressure. The white foam is triturated with pentane (50 mL), filtrated and dried under reduced pressure to afford pure iodinated compounds **4a-c**, respectively.

### General procedure for Sonogashira cross-coupling and subsequent desilylation

Iodinated nucleoside **4a-c** (5 mmol) was dissolved in a mixture of dry dimethyl formamide (DMF; 15 mL), dry triethyl amine (Et₃N; 2.06 mL, 3 eq.) and trimethyl silyl acetylene (2.06 mL, 3 eq.). Cuprous iodide (Cul; 190 mg, 0.2 eq.) and PdCl₂(PPh₃)₂ (350 mg, 0.1 eq.) were then added and the reaction mixture was stirred at room temperature until completion (typically 5-20 h, checked by TLC). Solvents were evaporated under reduced pressure. The oily residue was dissolved in AcOEt (250 mL) then washed with water (5 x 40 mL) and brine (40 mL). The organic layer was dried over MgSO₄ and the solvents were evaporated under reduced pressure to a dark oil. A first purification using a short path flash chromatography (eluent: methylene chloride (CH₂Cl₂) then MeOH/CH₂Cl₂ 5 %) afforded the desired compound contaminated with coloured reaction co-products. Pure silylated alkynes (2 mmol), obtained after a second flash chromatography (eluent: hexanes/AcOEt 7/3 then 1/1), were dissolved in dry CH₃CN (20 mL). Tetrabutyl ammonium fluoride (TBAF) monohydrate (583 mg, 1.05 eq.) was added and the resulting solution was stirred at room temperature until completion (typically 30 min. to 2 h, checked by TLC). Solvents were evaporated under reduced pressure at room temperature and the oily residue was submitted to a flash column chromatography (eluent: hexanes/AcOEt 1/1 then AcOEt then MeOH/AcOEt 2% then 5%) to afford pure 5-ethynyl nucleosides 5a-c, respectively.

### General procedure for mono-halogenation of 5-ethynyl nucleosides

5-Ethynyl nucleoside **5a-c** (0.5 mmol) is dissolved in 5 mL dry CH₃CN and the mixture is cooled in an ice bath. 0.7 mmol (1.4 eq.) of the halogenating reagent (i.e. IDCP (iodonium di-syn-collidine perchlorate) or Br(coll)₂ClO₄) and 11 mg Ag(coll)₂ClO₄ (0.05 eq.) are added and the mixture is stirred in the dark at rt (typically 2 h-20 h, checked by TLC). The reaction mixture is cooled to 0°C then quenched by a saturated Na₂S₂O₃ solution (2 mL) and extracted with AcOEt (4 x 10 mL). The organic layer is washed with water (10 mL), a 1 M HCl (hydrochloric acid) solution (3 x 5 mL), water (2 x 5 mL) and brine (10 mL) then dried over MgSO₄ and concentrated *in vacuo*. The solid residue is purified by flash chromatography (eluent: hexanes/AcOEt, 1/1, v/v) to yield the desired mono-halogenated compound **6-7a-c.**

### Example 1: 3',5'-Di-O-acetyl-5-(2-iodoethynyl)-2'-deoxyuridine (6a)

The title compound is obtained according to the above described general procedures from 3',5'-Di-*O*-acetyl-2'-deoxy-5-iodouridine (4a) (see Asakura, J. et al., *J. Org. Chem.,* **1990,** *55,* 4928-4933; and Asakura, J. et al., *Tetrahedron Lett*., **1988,** *29*, 2855-2858) or from 3',5'-di-*O-*acetyl-2'-deoxy-5-ethynyluridine (5a) (see US 5,028,596).
Yield: 93%.
mp: 175-177 (dec) °C
[*α*]_{D}²⁰-32 (c 0.5, CHCl₃)
¹H NMR (CDCl₃) δ 11.68 (br s, 1H, NH), 8.02 (s, 1H, H-6), 6.12 (t, J = 7.1 Hz, 1H, H-1'), 5.20 (m, 1H, H-3'), 4.33-4.15 (m, 3H, H-4',5'), 2.55 (m, 1H, H-2'a), 2.31 (m, 1H, H-2'b), 2.07 (s, 3H, OAc), 2.05 (s, 3H, OAc).
HRMS m/z 486.1982 calculated for C₁₅H₁₅IN₂O₇Na, found m/z 486.1986.

### Example 2: 3',5'-Di-O-acetyl-5-(2-bromoethynyl)-2'-deoxyuridine (7a)

According to a procedure similar to that of Example 1, the title compound is obtained as an oil.
Yield: 21%
[*α*]_{D}²⁰ -68 (c 1.0, CHCl₃)
¹H NMR (CDCl₃) *δ* 8.88 (br s, 1H, NH), 7.95 (s, 1H, H-6), 6.31 (dd, J = 7.3, 6.4 Hz, 1H, H-1'), 5.25 (m, 1H, H-3'), 4.38-4.30 (m, 3H, H-4',5'), 2.55 (m, 1H, H-2'a), 2.31 (m, 1H, H-2'b), 2.18 (s, 3H, OAc), 2.12 (s, 3H, OAc).
HRMS m/z 439.1978 calculated for C₁₅H₁₅BrN₂O₇Na, found m/z 439.1981.

Examples 3 to 6 below are obtained according to similar processes.

### Example 3: 2',3',5'-Tri-O-acetyl-5-(2-iodoethynyl)-uridine (6b)

Yield: 68%
mp: 100-102 °C
[*α*]_{D}²⁰-71 (c 1.0, CHCl₃)
¹H NMR (CDCl₃) *δ* 9.41 (br s, 1H, NH), 7.91 (s, 1H, H-6), 6.07 (d, *J* = 4.6 Hz, 1H, H-1'), 5.35 (m, 2H, H-2',3'), 4.38 (m, 3H, H-4',5'), 2.25 (s, 3H, OAc), 2.12 (s, 2 x 3H, 2 x OAc).
HRMS m/z 544.2352 calculated for C₁₇H₁₇IN₂O₉Na, found m/z 544.2349.

### Example 4: 2',3',5'-Tri-O-acetyl-5-(2-bromoethynyl)-uridine (7b)

Yield: 58% as a colorless oil
[*α*]_{D}²⁰ -59 (c 0.4, CHCl₃)
¹H NMR (CDCl₃) *δ* 9.75 (br s, 1H, NH), 7.92 (s, 1H, H-6), 6.07 (d, *J* = 4.4 Hz, 1H, H-1'), 5.35 (m, 2H, H-2',3'), 4.37 (m, 3H, H-4',5'), 2.22 (s, 3H, OAc), 2.12 (s, 2 x 3H, 2 x OAc).
HRMS m/z 497.2348 calculated for C₁₇H₁₇BrN₂O₉Na, found m/z 497.2351.

### Example 5: 2',5'-Di-O-acetyl-5-(2-iodoethynyl)-3'-deoxyuridine (6c)

*Step a):* 2',5'-Di-O-acetyl-3'-deoxy-5-iodouridine (4c)
   ¹H NMR (CDCl₃) δ 9.53 (br s, 1H, NH), 7.93 (s, 1H, H-6), 5.82 (d, *J* = 1.6 Hz, 1H, H-1'), 5.30 (m, 1H, H-2'), 4.55 (m, 1H, H-4'), 4.41 (dd, *J* = 12.8, 2.8 Hz, 1H, H-5'a), 4.33 (dd, *J* = 12.8, 3.8 Hz, 1H, H-5'b), 2.31-1.95 (m, 2H, H-3'), 2.23 (s, 3H, OAc), 2.13 (s, 3H, OAc).
*Step b):* 2',5'-Di-O-acetyl-3'-deoxy-5-ethynyluridine (5c)
   ¹H NMR (CDCl₃) *δ* 9.8 (br s, 1H, NH), 7.88 (s, 1 H, H-6), 5.78 (s, 1 H, H-1'), 5.31 (d, *J* = 7.5 Hz, 1H, H-2'), 4.48 (m, 1H, H-4'), 4.35 (dd, *J* = 12.5, 1.8 Hz, 1H, H-5'a), 4.25 (dd, J = 12.5, 3.4 Hz, 1 H, H-5'b), 3.1 (s, 1 H, H-Csp), 2.31-1.95 (m, 2H, H-3'), 2.12 (s, 3H, OAc), 2.05 (s, 3H, OAc)
   HRMS m/z 360.2968 calculated for C₁₅H₁₆N₂O₇Na, found m/z 360.2971.
*Step c):* 2',5'-Di-O-acetyl-5-(2-iodoethynyl)-3'-deoxyuridine (6c)
   Yield: 76% as an oil
   [*α*]_{D}²⁰-49 (c 0.8, CHCl₃)
   ¹H NMR (CDCl₃) *δ* 11.68 (br s, 1H, NH), 7.98 (s, 1H, H-6), 5.74 (s, 1H, H-1'), 5.30 (d, *J* = 7.5 Hz, 1H, H-2'), 4.43 (m, 1H, H-4'), 4.28 (br s, 2H, H-5'), 2.31-1.95 (m, 2H, H-3'), 2.09 (s, 3H, OAc), 2.05 (s, 3H, OAc).
   HRMS m/z 486.1982 calculated for C₁₅H₁₅IN₂O₇Na, found m/z 486.1986.

### Example 6: 2',5'-Di-O-acetyl-5-(2-bromoethynyl)-3'-deoxyuridine (7c)

Yield: 25% as an oil
[*α*]_{D}²⁰ -52 (c 0.4, CHCl₃)
¹H NMR (DMSO-*d*_{*6*}) *δ* 11.75 (br s, 1H, NH), 8.07 (s, 1H, H-6), 5.85 (s, 1H, H-1'), 5.31 (d, *J* = 7.5 Hz, 1H, H-2'), 4.43 (m, 1H, H-4'), 4.28 (br s, 2H, H-5'), 2.31-1.95 (m, 2H, H-3'), 2.08 (s, 3H, OAc), 2.06 (s, 3H, OAc).
HRMS m/z 439.1978 calculated for C₁₅H₁₅BrN₂O₇Na, found m/z 439.1975.

### General procedure for di-halogenation of 5-ethynyl nucleosides

5-ethynyl nucleoside **5a-c** (0.5 mmol) is dissolved in 5 mL dry CH₃CN and the mixture is cooled in an ice bath. 0.6 mmol (1.2 eq.) of a solution of the halogenating reagent (i.e. I₂, IBr, ICI or Br₂) in 1 mL dry CH₃CN is added dropwise and the mixture is stirred at 0°C until completion (typically 15 min.-2 h, checked by TLC). The reaction mixture is quenched at 0°C by a saturated Na₂S₂O₃ solution (2 mL) then extracted with AcOEt (3 x 10 mL). The organic layer is washed with water (2 x 5 mL) and brine (10 mL) then dried over MgSO₄ and concentrated *in vacuo* to yield the desired di-halogenated compounds of examples 7-18 (**8-11a-c**) in a reasonably pure form. Pure analytical samples were obtained using flash chromatography (eluent: hexanes/AcOEt, 1/1, v/v).

### Example 7: (E)-3',5'-Di-O-acetyl-5-(1,2-diiodovinyl)-2'-deoxyuridine (8a)

94% yield; mp 76-78 °C; [*α*]_{D}²⁰ -19 (c 0.3, CHCl₃); ¹H NMR (CDCl₃) *δ* 9.72 (br s, 1H, NH), 7.67 (s, 1H, H-6), 7.45 (s, 1H, =CHI), 6.36 (dd, *J* = 8.1, 5.6 Hz, 1H, H-1'), 5.25 (m, 1H, H-3'), 4.50-4.28 (m, 3H, H-4',5'), 2.60 (m, 1H, H-2'a), 2.30 (m, 1H, H-2'b), 2.17 (s, 3H, OAc), 2.08 (s, 3H, OAc). HRMS m/z 614.1106 calculated for C₁₅H₁₆I₂N₂O₇Na, found m/z 614.1110.

### Example 8: (E)-2',3',5'-Tri-O-acetyl-5-(1,2-diiodovinyl)-uridine (8b)

98% yield; mp 83-85 °C; [*α*]_{D}²⁰ -41 (c 1.0, CHCl₃); ¹H NMR (CDCl₃) *δ* 9.56 (br s, 1 H, NH), 7.58 (s, 1H, H-6), 7.40 (s, 1H, =CHI), 6.15 (d, J = 4.6 Hz, 1H, H-1'), 5.36 (m, 2H, H-2',3'), 4.38 (br s, 3H, H-4',5'), 2.19 (s, 3H, OAc), 2.14 (s, 3H, OAc), 2.11 (s, 3H, OAc). HRMS m/z 672.1476 calculated for C₁₇H₁₈I₂N₂O₉Na, found m/z 672.1480.

### Example 9: (E)-2',5'-Di-O-acetyl-5-(1,2-diiodovinyl)-3'-deoxyuridine (8c)

87% yield; mp 75-77 °C; [*α*]_{D}²⁰ -23 (c 0.3, CHCl₃); ¹H NMR (CDCl₃) *δ* 9.38 (br s, 1H, NH), 7.64 (s, 1H, H-6), 7.39 (s, 1H, =CHI), 5.92 (d, *J* = 1.6 Hz, 1H, H-1'), 5.38 (m, 1H, H-4'), 4.58 (m, 1H, H-2'), 4.44 (dd, *J* = 12.5, 4.4 Hz, 1H, H-5'a), 4.33 (dd, 1H, H-5'b), 2.31-1.95 (m, 2H, H-3'), 2.14 (s, 3H, OAc), 2.12 (s, 3H, OAc). HRMS m/z 614.1106 calculated for C₁₅H₁₆I₂N₂O₇Na, found m/z 614.1101.

### Example 10: (E)-3',5'-Di-O-acetyl-5-(1-bromo-2-iodovinyl)-2'-deoxyuridine (9a)

96% yield; mp 82-84 °C; [α]_{D}²⁰ -14 (c 4.0, CHCl₃); ¹H NMR (CDCl₃) *δ* 9.78 (br s, 1H, NH), 7.71 (s, 1H, H-6), 7.08 (s, 1H, =CHI), 6.25 (dd, *J* = 8.1, 5.6 Hz, 1H, H-1'), 5.28 (m, 1H, H-3'), 4.50-4.05 (m, 3H, H4', 5'), 2.58 (m, 1H, H-2'a), 2.20 (m, 1H, H-2'b), 2.12 (s, 3H, OAc), 1.96 (s, 3H, OAc). HRMS m/z 567.1102 calculated for C₁₅H₁₆BrIN₂O₇Na, found m/z 567.1107.

### Example 11: (E)-2',3',5'-Tri-O-acetyl-5-(1-bromo-2-iodovinyl)-uridine (9b)

92% yield; mp 86-88 °C; [α]_{D}²⁰ -39 (c 2.0, CHCl₃); ¹H NMR (CDCl₃) *δ* 9.57 (br s, 1 H, NH), 7.66 (s, 1H, H-6), 7.15 (s, 1H, =CHI), 6.15 (d, *J* = 4.7 Hz, 1H, H-1'), 5.38 (m, 2H, H-2',3'), 4.38 (m, 3H, H-4',5'), 2.18 (s, 3H, OAc), 2.15 (s, 3H, OAc), 2.13 (s, 3H, OAc). HRMS m/z 625.1469 calculated for C₁₇H₁₈BrIN₂O₉Na, found m/z 625.1473.

### Example 12: (E)-2',5'-Di-O-acetyl-5-(1-bromo-2-iodovinyl)-3'-deoxyuridine (9c)

89% yield as an oil; [α]_{D}²⁰ -19 (c 0.5, CHCl₃); ¹H NMR (CDCl₃) *δ* 9.63 (br s, 1H, NH), 7.61 (s, 1 H, H-6), 7.13 (s, 1H, =CHI), 5.92 (s, 1 H, H-1'), 5.38 (m, 1H, H-4'), 4.58 (m, 1H, H-2'), 4.44 (dd, *J* = 12.4, 2.6 Hz, 1H, H-5'a), 4.33 (dd, *J* = 12.4, 4.4 Hz, 1H, H-5'b), 2.31-1.95 (m, 2H, H-3'), 2.16 (s, 3H, OAc), 2.14 (s, 3H, OAc). HRMS m/z 567.1102 calculated for C₁₅H₁₆BrIN₂O₇Na, found m/z 567.1105.

### Example 13: (E)-3',5'-Di-O-acetyl-5-(1-chloro-2-iodovinyl)-2'-deoxyuridine (10a)

91% yield; mp 81-83 °C; [α]_{D}²⁰ -14 (c 0.7, CHCl₃); ¹H NMR (CDCl₃) *δ* 9.28 (br s, 1H, NH), 7.75 (s, 1H, H-6), 6.91 (s, 1H, =CHI), 6.32 (m, 1H, H-1'), 5.24 (m, 1H, H-3'), 4.50-4.30 (m, 3H, H-4',5'), 2.75 (m, 1H, H-2'a), 2.18 (m, 1 H, H-2'b), 2.13 (s, 2 x 3H, 2 x OAc). HRMS m/z 522.6592 calculated for C₁₅H₁₆ClIN₂O₇Na, found m/z 522.6597.

### Example 14: (E)-2',3',5'-Tri-O-acetyl-5-(1-chloro-2-iodovinyl)-uridine (10b)

97% yield; mp 85-87 °C; [α]_{D}²⁰ -27 (c 0.8, CHCl₃); ¹H NMR (CDCl₃) *δ* 9.43 (br s, 1H, NH), 7.68 (s, 1H, H-6), 6.92 (s, 1H, =CHI), 6.15 (br s, 1H, H-1'), 5.36 (m, 2H, H-2',3'), 4.38 (m, 3H, H-4',5'), 2.17 (s, 3H, OAc), 2.14 (s, 3H, OAc), 2.12 (s, 3H, OAc). HRMS m/z 580.6962 calculated for C₁₇H₁₈CllN₂O₉Na, found m/z 580.6958.

### Example 15: (E)-2',5'-Di-O-acetyl-5-(1-chloro-2-iodovinyl)-3'-deoxyuridine (10c)

83% yield; mp 87-89 °C; [α]_{D}²⁰ -13 (c 1.0, CHCl₃); ¹H NMR (CDCl₃) *δ* 9.75 (br s, 1H, NH), 7.74 (s, 1H, H-6), 6.90 (s, 1H, =CHI), 5.91 (d, *J* = 1.7 Hz, 1H, H-1'), 5.38 (m, 1H, H-4'), 4.58 (m, 1H, H-2'), 4.43 (dd, *J* = 12.5, 2.8 Hz, 1H, H-5'a), 4.33 (dd, *J* = 12.5, 4.1 Hz, 1H, H-5'b), 2.35-1.95 (m, 2H, H-3'), 2.14 (s, 2 x 3H, 2 x OAc). HRMS m/z 522.6592 calculated for C₁₅H₁₆CIIN₂O₇Na, found m/z 522.6593.

### Example 16: 3',5'-Di-O-acetyl-5-(1,2-dibromovinyl)-2'-deoxyuridine (11a)

97% yield; mp 79-81 °C; [*α*]_{D}²⁰ -15 (c 1.0, CHCl₃); ¹H NMR (CDCl₃) *δ* 9.85 (br s, 1H, NH), 7.75 (s, 1H, H-6), 6.89 (s, 1H, =CHBr), 6.33 (dd, *J* = 8.1, 5.6 Hz, 1 , H-1'), 5.24 (m, 1H, H-3'), 4.45-4.25 (m, 3H, H-4',5'), 2.65 (m, 1H, H-2'a), 2.18 (m, 1H, H-2'b), 2.14 (s, 2 x 3H, 2 x OAc). HRMS m/z 520.1098 calculated for C₁₅H₁₆Br₂N₂O₇Na, found m/z 520.1099.

### Example 17: (E)-2',3',5'-Tri-O-acetyl-5-(1,2-dibromovinyl)-uridine (11b)

92% yield; mp 83-85 °C; [*α*]_{D}²⁰ -43 (c 0.5, CHCl₃); ¹H NMR (CDCl₃) *δ* 9.38 (br s, 1H, NH), 7.66 (s, 1H, H-6), 6.90 (s, 1H, =CHBr), 6.12 (d, *J* = 4.8 Hz, 1H, H-1'), 5.36 (m, 2H, H-2',3'), 4.38 (m, 3H, H-4',5'), 2.17 (s, 3H, OAc), 2.14 (s, 3H, OAc), 2.12 (s, 3H, OAc). HRMS m/z 578.1468 calculated for C₁₇H₁₈Br₂N₂O₉Na, found m/z 578.1473.

### Example 18: 2',5'-Di-O-acetyl-5-(1,2-dibromovinyl)-3'-deoxyuridine (11c)

82% yield; mp 78-80 °C; [*α*]_{D}²⁰ -16 (c 0.8, CHCl₃); ¹H NMR (CDCl₃) *δ* 9.61 (br s, 1H, NH), 7.72 (s, 1H, H-6), 6.88 (s, 1H, =CHI), 5.88 (d, J = 1.5 Hz, 1H, H-1'), 5.38 (m, 1H, H-4'), 4.58 (m, 1H, H-2'), 4.43 (dd, *J* = 12.5, 2.8 Hz, 1H, H-5'a), 4.33 (dd, *J* = 12.5, 4.1 Hz, 1H, H-5'b), 2.35-1.95 (m, 2H, H-3'), 2.15 (s, 3H, OAc), 2.14 (s, 3H, OAc). HRMS m/z 520.1098 calculated for C₁₅H₁₆Br₂N₂O₇Na, found m/z 520.1103.

### General procedure for deacetylation

Acetylated nucleoside analogue **6-11a-c** (1 mmol) is dissolved in 10 mL pyridine and 5 mL EtOH. The reaction mixture is cooled to -10°C and 5 mL of a 1M NaOH (sodium hydroxide) aqueous solution is added. The resulting solution is stirred at this temperature until completion (typically 1-4 h, checked by TLC). The reaction mixture is neutralized with Dowex then filtered through a fritted glass funnel. Solvents are evaporated *in vacuo* and the oily residue is submitted to a flash column chromatography using an appropriate eluent (typically hexanes/AcOEt 25/75 then AcOEt then MeOH/AcOEt 1 %) to yield pure compounds **12-17a-c** (Examples 19-36).

### Example 19: 5-(2-Iodoethynyl)-2'-deoxyuridine (12a)

68% yield; mp 135-137 °C; [*α*]_{D}²⁰ +14 (c 0.8, MeOH); ¹H NMR (CD₃OD) *δ* 8.32 (s, 1H, H-6), 6.22 (t, *J* = 6.6 Hz, 1H, H-1'), 4.41 (m, 1H, H-3'), 3.95 (m, 1H, H-4'), 3.83 (dd, *J* = 12.1, 3.0 Hz, 1H, H-5'a), 3.73 (dd, *J* = 12.1, 3.4 Hz, 1H, H-5'b), 2.40-2.12 (m, 2H, H-2'). HRMS m/z 402.1229 calculated for C₁₁H₁₁IN₂O₅Na, found m/z 402.1231.

### Example 20: 5-(2-Iodoethynyl)-uridine (12b)

70% yield as an oil; [*α*]_{D}²⁰ -12 (c 0.2, MeOH); ¹H NMR (DMSO-d6) *δ* 11.65 (br s, 1H, NH), 8.29 (s, 1H, H-6), 5.72 (m, 1H, H-1'), 5.05-5.12 (3 x br s, 3 x OH), 4.05-3.95 (m, 2H, H-2',3'), 3.85 (m, 1H, H-4'), 3.70-355 (m, 2H, H-5'). HRMS m/z 418.1223 calculated for C₁₁H₁₁IN₂O₆Na, found m/z 418.1219.

### Example 21: 5-(2-Iodoethynyl)-3'-deoxyuridine (12c)

66% yield as an oil; [*α*]_{D}²⁰ +6 (c 1.0, MeOH); ¹H NMR (DMSO-*d*_{*6*}) *δ* 11.65 (s, 1H, NH), 8.48 (s, 1H, H-6), 5.59 (s, 1H, H-1'), 5.54 (d, *J* = 4.1 Hz, 1H, OH), 5.23 (t, *J* = 5.1 Hz, 1H, OH), 4.32 (m, 1H, H-4'), 4.21 (m, 1H, H-2'), 3.75 (m, 1H, H-5'a), 3.52 (m, 1H, H-5'b), 1.98 (m, 1H, H-3'a), 1.76 (m, 1H, H-3'b). HRMS m/z 402.1229 calculated for C₁₁H₁₁IN₂O₅Na, found m/z 402.1233.

### Example 22: 5-(2-Bromoethynyl)-2'-deoxyuridine (13a)

80% yield as an oil; [*α*]_{D}²⁰ +3 (c 0.3, MeOH); ¹H NMR (CD₃OD) *δ* 8.30 (s, 1H, H-6), 6.05 (t, J = 6.3 Hz, 1H, H-1'), 4.21 (m, 1H, H-3'), 3.78 (m, 1H, H-4'), 3.65-3.50 (m, 2H, H-5'), 2.15 (dd, *J* = 5.6, 5.0 Hz, 2H, H-2'). HRMS m/z 355.1225 calculated for C₁₁H₁₁BrN₂O₅Na, found m/z 355.1227.

### Example 23: 5-(2-bromoethynyl)-uridine (13b)

74% yield as an oil; [*α*]_{D}²⁰ -23 (c 0.5, MeOH); ¹H NMR (DMSO-d6) *δ* 11.69 (br s, 1H, NH), 8.39 (s, 1H, H-6), 5.73 (d, J = 4.7 Hz, 1H, H-1'), 5.41 (d, *J* = 5.3 Hz, OH), 5.24 (t, *J* = 4.7 Hz, OH), 5.08 (d, *J* = 5.3 Hz, OH), 4.10-3.91 (m, 2H, H-2',3'), 3.85 (m, 1H, H-4'), 3.75-350 (m, 2H, H-5'). HRMS m/z 371.1219 calculated for C₁₁H₁₁BrN₂O₆Na, found m/z 371.1224.

### Example 24: 5-(2-Bromoethynyl)-3'-deoxyuridine (13c)

72% yield as an oil; [*α*]_{D}²⁰ -29 (c 1.0, MeOH); ¹H NMR (DMSO-*d*_{*6*}) *δ* 11.63 (s, 1H, NH), 8.53 (s, 1H, H-6), 5.59 (s, 1H, H-1'), 5.56 (d, *J* = 4.1 Hz, 1H, OH), 5.26 (t, *J* = 5.1 Hz, 1 H, OH), 4.32 (m, 1H, H-4'), 4.25 (m, 1H, H-2'), 3.81 (m, 1H, H-5'a), 3.61 (m, 1H, H-5'b), 1.95 (m, 1H, H-3'a), 1.72 (m, 1H, H-3'b). HRMS m/z 355.1225 calculated for C₁₁H₁₁BrN₂O₅Na, found m/z 355.1223.

### Example 25: (E)-5-(1,2-Diiodovinyl)-2'-deoxyuridine (14a)

72% yield; mp 103-105 °C; [*α*]_{D}²⁰ +28 (c 0.3, MeOH); ¹H NMR (CD₃OD) *δ* 8.27 (s, 1H, H-6), 7.52 (s, 1H, =CHI), 6.28 (t, *J* = 6.3 Hz, 1H, H-1'), 4.43 (m, 1H, H-3'), 3.97 (m, 1H, H-4'), 3.83 (dd, *J =* 11.9, 3.2 Hz, 1H, H-5'a), 3.73 (m, *J =* 11.9, 3.4 Hz, 1H, H-5'b), 2.43-2.12 (m, 2H, H-2'). HRMS m/z 530.0353 calculated for C₁₁H₁₂I₂N₂O₅Na, found m/z 530.0558.

### Example 26: (E)-5-(1,2-Diiodovinyl)-uridine (14b)

82% yield; mp 107-109 °C; [*α*]_{D}²⁰ +5 (c 1.0, MeOH); ¹H NMR (DMSO-d6) *δ* 11.65 (br s, 1H, NH), 8.05 (s, 1H, H-6), 7.05 (s, IH, =CHI), 5.80 (d, *J* =4.7 Hz, 1H, H-1'), 5.50-5.10 (3 x br s, 3 x OH), 4.12-3.95 (m, 2H, H-2',3'), 3.87 (m, 1H, H-4'), 3.75-355 (m, 2H, H-5'). HRMS m/z 546.0347 calculated for C₁₁H₁₂I₂N₂O₆Na, found m/z 546.0351.

### Example 27: (E)-5-(1,2-Diiodovinyl)-3'-deoxyuridine (14c)

76% yield as an oil; [*α*]_{D}²⁰ + 1.0 (c 0.8, MeOH); ¹H NMR (DMSO-*d*_{*6*} + D₂O) δ 8.21 (s, 1H, H-6), 7.55 (s, 1H, =CHI), 5.61 (s, 1H, H-1'), 4.32 (m, 1H, H-4'), 4.25 (m, 1H, H-2'), 3.81 (m, 1H, H-5'a), 3.52 (m, 1H, H-5'b), 1.97 (m, 1H, H-3'a), 1.74 (m, 1H, H-3'b). HRMS m/z 530.0553 calculated for C₁₁H₁₂I₂N₂O₅Na, found m/z 530.0554.

### Example 28: (E)-5-(1-Bromo-2-iodovinyl)-2'-deoxyuridine (15a)

78% yield as an oil; [*α*]_{D}²⁰ +26 (c 2.0, MeOH); ¹H NMR (CD₃OD) *δ* 8.35 (s, 1H, H-6), 7.39 (s, 1H, =CHI), 6.25 (t, *J* = 6.5 Hz, 1H, H-1'), 4.45 (m, 1H, H-3'), 3.94 (m, 1H, H-4'), 3.86 (dd, *J* = 11.9, 3.2 Hz, 1H, H-5'a), 3.72 (dd, *J* = 11.9, 3.4 Hz, 1H, H-5'b), 2.45-2.15 (m, 2H, H-2'). HRMS m/z 483.0349 calculated for C₁₁H₁₂BrIN₂O₅Na, found m/z 483.0352.

### Example 29: (E)-5-(1-Bromo-2-iodovinyl)-uridine (15b)

76% yield as an oil; [*α*]_{D}²⁰ -1 (c 0.3, MeOH); ¹H NMR (D₂O) δ 8.27 (s, 1H, H-6), 7.45 (s, 1H, =CHI), 5.79 (d, *J* = 4.7 Hz, 1H, H-1'), 4.15-3.92 (m, 2H, H-2',3'), 3.88 (m, 1H, H-4'), 3.67 (dd, *J* = 11.8, 2.5 Hz, 1H, H-5'a), 3.56 (dd, 1H, H-5'b). HRMS m/z 499.0343 calculated for C₁₁H₁₂BrIN₂O₆Na, found m/z 499.0348.

### Example 30: (E)-5-(1-Bromo-2-iodovinyl)-3'-deoxyuridine (15c).

68% yield as an oil; [*α*]_{D}²⁰ +3 (c 1.0, MeOH); ¹H NMR (DMSO-*d*_{*6*} + D₂O) *δ* 8.35 (s, 1H, H-6), 7.39 (s, 1H, =CHI), 5.61 (d, *J* = 1.3 Hz, 1H, H-1'), 4.32 (m, 1H, H-4'), 4.25 (m, 1H, H-2'), 3.76 (dd, *J* = 12.2, 2.4 Hz, 1H, H-5'a), 3.52 (dd, *J* = 12.2, 2.8 Hz, 1H, H-5'b), 1.97 (m, 1H, H-3'a), 1.75 (m, 1H, H-3'b). HRMS m/z 483.0349 calculated for C₁₁H₁₂BrIN₂O₅Na, found m/z 483.0344.

### Example 31: (E)-5-(1-Chloro-2-iodovinyl)-2'-deoxyuridine (16a)

71% yield; mp 109-111 °C; [*α*]_{D}²⁰ +34 (c 0.5, MeOH); ¹H NMR (CD₃OD) *δ* 8.38 (s, 1H, H-6), 7.06 (s, 1H, =CHI), 6.30 (t, *J* = 6.5 Hz, 1H, H-1'), 4.43 (ddd, *J* = 6.5, 3.8, 3.6 Hz, 1H, H-3'), 3.96 (ddd, *J* = 3.6, 3.4, 3.0 Hz, 1H, H-4'), 3.83 (dd, *J* = 11.9, 3.1 Hz, 1H, H-5'a), 3.73 (dd, *J* = 11.9, 3.4 Hz, 1H, H-5'b), 2.45-2.10 (m, 2H, H-2'). HRMS m/z 438.5839 calculated for C₁₁H₁₂CIIN₂O₅Na, found m/z 438.5843.

### Example 32: (E)-5-(1-Chloro-2-iodovinyl)-uridine (16b)

78% yield; mp 109-111 °C; [*α*]_{D}²⁰ +5 (c 0.4, MeOH); ¹H NMR (D₂O) *δ* 8.45 (s, 1H, H-6), 7.05 (s, IH, =CHI), 5.89 (br s, 1H, H-1'), 4.30 (br s, 2H, H-2',3'), 4.10 (m, 1H, H-4'), 3.85 (dd, *J* = 11.5, 2.2 Hz, 1H, H-5'a), 3.75 (dd, 1H, H-5'b). HRMS m/z 454.5833 calculated for C₁₁H₁₂ICIN₂O₆Na, found m/z 545.5835.

### Example 33: (E)-5-(1-Chloro-2-iodovinyl)-3'-deoxyuridine (16c)

70% yield as an oil; [*α*]_{D}²⁰ +2 (c 2.0, MeOH); ¹H NMR (DMSO-*d*_{*6*} + D₂O) δ 8.38 (s, 1H, H-6), 7.18 (s, 1H, =CHI), 5.65 (d, *J* = 1.2 Hz, 1H, H-1'), 4.32 (m, 1H, H-4'), 4.25 (m, 1H, H-2'), 3.76 (dd, *J* = 12.3, 2.6 Hz, 1H, H-5'a), 3.52 (dd, *J* = 12.3, 2.8 Hz, 1H, H-5'b), 2.01 (m, 1H, H-3'a), 1.75 (m, 1H, H-3'b). HRMS m/z 438.5839 calculated for C₁₁H₁₂CIIN₂O₅Na, found m/z 438.5837.

### Example 34: (E)-5-(1,2-Dibromovinyl)-2'-deoxyuridine (17a)

85% yield as an oil; [*α*]_{D}²⁰ +26 (c 0.5, MeOH); ¹H NMR (CD₃OD) δ 8.39 (s, 1H, H-6), 7.06 (s, 1H, =CHBr), 6.28 (t, *J* = 6.5 Hz, 1H, H-1'), 4.41 (m, 1H, H-3'), 3.95 (m, 1H, H-4'), 3.82 (dd, *J* = 11.9, 2.9 Hz, 1H, H-5'a), 3.73 (dd, *J* = 11.9, 3.4 Hz, 1H, H-5'b), 2.45-2.10 (m, 2H, H-2'). HRMS m/z 436.0345 calculated for C₁₁H₁₂Br₂N₂O₅Na, found m/z 436.0348.

### Example 35: (E)-5-(1,2-Dibromovinyl)-uridine (17b)

72% yield as an oil; [*α*]_{D}²⁰ -14 (c 0.5, MeOH); ¹H NMR (D₂O) δ 8.45 (s, 1H, H-6), 7.07 (s, 1H, =CHBr), 5.93 (d, J = 5.2 Hz, 1H, H-1'), 4.18 (m, 2H, H-2',3'), 4.05 (m, 1H, H-4'), 3.88 (dd, *J* = 12.5, 2.5 Hz, 1H, H-5'a), 3.74 (dd, *J* = 12.5, 2.8 Hz, 1H, H-5'b). HRMS m/z 452.0339 calculated for C₁₁H₁₂Br₂N₂O₆Na, found m/z 452.0342.

### Example 36: (E)-5-(1,2-Dibromovinyl)-3'-deoxyuridine (17c)

74% yield as an oil; [*α*]_{D}²⁰ +5 (c 1.0, MeOH); ¹H NMR (DMSO-*d*_{*6*} + D₂O) *δ* 8.42 (s, 1H, H-6), 7.25 (s, 1H, =CHI), 5.64 (br s, 1H, H-1'), 4.35 (m, 1H, H-4'), 4.25 (m, 1H, H-2'), 3.78 (m, H-5'a), 3.54 (m, H-5'b), 1.95 (m, 1H, H-3'a), 1.75 (m, 1H, H-3'b). HRMS m/z 436.0345 calculated for C₁₁H₁₂Br₂N₂O₅Na, found m/z 436.0340.

### Preparation of the key intermediate 18 (for both 3'-F and 3'-N₃ series)

### 1-(4-Hydroxy-5-trityloxymethyltetrahydrofuran-2-yl)-1H-pyrimidine-2,4-dione (32)

A solution of 2'-deoxyuridine (10.0 g, 43.9 mmol) and para-anisylchlorodiphenylmethane (16.3 g, 52.6 mmol) in dry pyridine (235 mL) is refluxed for 2 hours. After cooling at room temperature (rt), pyridine is evaporated under reduced pressure, then dichloromethane (400 mL) is added and the solution is washed with water (50 mL x 3), dried over MgSO₄, filtered through a fritted glass funnel and concentrated under reduced pressure to afford the crude product, which is submitted to a flash silica gel column chromatography (eluent: hexanes/EtOAc: 5/5 to EtOAc/MeOH: 9/1) to give the pure protected nucleoside 32 (21.9 g, 99 %) as a white solid. The physico-chemical data of the compound are fully related with those previously published.

### 1-(4-Hydroxy-5-trityloxymethyltetrahydrofuran-2-yl)-5-iodo-1H-pyrimidine-2,4-dione (33)

A solution of **32** (21.9 g, 43.8 mmol), I₂ (17.0 g, 66.9 mmol), CAN (17.7 g, 32.3 mmol), NaHCO₃ (1.0 g) in dry acetonitrile (480 mL) is refluxed for **4** days. After cooling at rt, a solution of saturated Na₂SO₃ (800 mL) and EtOAc (200 mL) are added: aqueous layer is extracted with EtOAc (300 mL x2), then organic layer is washed with saturated Na₂SO₃ (300 mL), dried over MgSO₄, filtered through a fritted glass funnel and concentrated under reduced pressure to afford the crude product, which is submitted to a flash silica gel column chromatography (eluent: hexanes/EtOAc: 6/4 to EtOAc) to give the pure iodinated nucleoside 33 (12.8 g, 57 %) as a white solid. The physico-chemical data of the compound are fully related with those previously published.

### 1-(4-Hydroxy-5-trityloxymethyl-tetrahydro-furan-2-yl)-5-trimethylsilanylethynyl-1H-pyrimidine-2,4-dione (34)

**33** (5.00 g, 8.0mmol) is dissolved in a mixture of dry DMF (62.5 mL), dry Et₃N (3.3 mL, 24.0 mmol), and trimethylsilylacetylene (3.4 mL, 24.0 mmol). Cul (300.0 mg, 1.6 mmol) and PdCl₂(PPh₃)₂ (563 mg) are then added and the reaction mixture is stirred at rt under argon for 16 hours. Solvents are evaporated under reduced pressure. The crude product is submitted to a flash silica gel column chromatography (eluent: hexanes/EtOAc: 5/5 to 2/8) to give **34** (4.10 g, 86 %) as a pale yellow solid. [*α*]_{D}²⁰ +27.5 (c 0.5, CHCl₃); mp 104 °C; ¹H NMR (CDCl₃) δ: 2.08-2.33 (m, 1H, H-2'a), 2.42-2.64 (m, 1H, H-2'b), 3.20-3.52 (m, 2H, H-5'), 3.78 (s, 3H, OCH3), 4.05-4.25 (m, 1H, H-4'), 4.40-4.53 (m, 1H, H-3'), 6.31 (dd, *J* = 5.6 Hz, 8.1 Hz, 1H, H-1'), 6.78-7.55 (m, 14H, trityl), 8.05 (s, 1H, H-6); ¹³C NMR (CDCl₃) *δ*: 41.5 (C-2'), 55.3 (OCH3), 63.7 (C-5'), 72.5 (C-3'), 85.9 (C-1'), 86.6 (C-4'), 87.3 (C), 94.9 (C), 99.9 (C), 100.7 (C), 113.5 (CH), 127.2 (CH), 128.2 (CH), 128.3 (C), 130.5 (CH), 135.1 (C), 142.8 (C-6), 144.0 (C), 144.1 (C), 149.5 (C=O), 158.8 (C), 161.5 (C);

### 5-Ethynyl-1-(4-hydroxy-5-trityloxymethyl-tetra hydro-furan-2-yl)-1H-pyrimidine-2,4-dione (35)

A solution of **34** (616.3 mg, 1.03mmol), TBAF (303.4 mg, 1.16 mmol) in dry acetonitrile (24.0 mL) is stirred at rt for 2 hours. Solvent is removed under reduced pressure, the crude product is submitted to a flash silica gel column chromatography (eluent: hexanes/EtOAc: 5/5 to 2/8) to give **35** (516.8 mg, 95 %) as a pale yellow solid. [*α*]_{D}²⁰ +14.5 (c 1.0, CHCl₃); mp 110°C; ¹H NMR (CDCl₃) *δ*: 2.22-2.40 (m, 1H, H-2'a), 2.45-2.60 (m, 1H, H-2'b), 2.90 (s, 1H, CH alkyne), 3.41 (t, *J =* 3.6 Hz, 2H, H-5'), 3.81 (s, 3H, OCH3), 4.15-4.20 (m, 1H, H-4'), 4.51-4.62 (m, 1H, H-3'), 6.30 (t, *J* = 8.2 Hz, 1H, H-1'), 6.70-7.50 (m, 14H, trityl), 8.10 (s, 1H, H-6); ¹³C NMR (CDCl₃) *δ*: 41.6 (C-2'), 55.4 (OCH3), 63.7 (C-5'), 72.4 (C-3'), 74.2 (C), 82.2 (C), 85.9 (C-1'), 86.6 (C-4'), 87.5 (C), 99.4 (C), 113.5 (CH), 127.3 (CH), 128.2 (CH), 128.3 (C), 128.4 (C), 130.5 (CH), 135.1 (C), 143.8 (C-6), 143.9 (C), 144.1 (C), 149.4 (C=O), 158.9 (C), 161.7 (C).

### Methanesulfonic acid 5-(5-ethynyl-2,4-dioxo-3,4-dihydro-2H-pyrimidin-1-yl)-2-trityloxy-methyltetrahydrofuran-3-yl ester (18)

A solution of **35** (500.0 mg, 0.95 mmol) and methanesulfonylchloride (0.73 mL, 9.50 mmol) in dry pyridine at 0°C is allowed to warm to rt and is stirred for 16 hours. After cooling at 0°C, ice, water (100 mL) and EtOAc (100 mL) are added, the aqueous layer is extracted with EtOAc (50 mL x3), the organic layers are washed with water (50 mL x3), dried over MgSO₄, filtered through a fritted glass funnel and concentrated under reduced pressure to afford the crude product, which is submitted to a flash silica gel column chromatography (eluent: hexanes/EtOAc: 5/5) to give **18** (587.0 mg, 99 %) as an orange solid. [*α*]_{D}²⁰ +28.5 (c 1.0, CHCl₃); mp 99 °C; ¹H NMR (CDCl₃) *δ*: 2.30-2.50 (m, 1H, H-2'a), 2.70-2.82 (m, 1H, H-2'b), 2.91 (s, 1H, CH alkyne), 3.02 (s, 3H, SCH₃), 3.33-3.57 (m, 2H, H-5'), 3.80 (s, 3H, OCH₃), 4.35 (se, 1H, H-4'), 5.31 (se, 1H, H-3'), 6.29 (t, *J* = 8.2 Hz, 1H, H-1'), 6.75-7.50 (m, 14H, trityl), 8.07 (s, 1H, H-6), 8.58 (brs, 1H, NH); ¹³C NMR (CDCl₃) *δ*: 38.8 (SCH₃), 39.3 (C-2'), 55.4 (OCH₃), 63.0 (C-5'), 74.0 (C-3'), 79.8 (C), 82.4 (C), 84.5 (C-1'), 85.4 (C-4'), 88.0 (C), 99.9 (C), 113.6 (CH), 127.5 (CH), 128.3 (CH), 128.3 (CH), 129.2 (C), 130.5 (CH), 134.6 (C), 143.2 (C-6), 143.6 (C), 143.7 (C), 149.2 (C=O), 159.0 (C), 161.2 (C);

### Example 37: 5-(1,2-Di-iodovinyl)-1-(4-fluoro-5-hydroxymethyltetrahydrofuran-2-yl)-1H-pyrimidine-2,4-dione (25a)

Step a): 5-Ethynyl-1-(4-hydroxy-5-trityloxymethyltetrahydrofuran-2-yl)-1*H*-pyrimidine-2,4-dione (**19**)
   A solution of **18** (50.0 mg, 0.08 mmol) in dioxanne (2.0 mL) and aqueous 1M NaOH (168 µL, 0.16 mmol) is refluxed for 2 hours. After cooling at rt, solvents are removed in vaccuo and the crude product is submitted to a flash silica gel column chromatography (eluent: hexanes/EtOAc: 5/5 to EtOAc) to give **19** (41.0 mg, 93 %) as a pale yellow solid. [*α*]_{D}²⁰ -13.0 (c 1.0, CHCl₃); mp 124 °C; ¹H NMR (CDCl₃) *δ*: 2.08-2.25 (m, 1H, H-2'a), 2.47-2.66 (m, 1H, H-2'b), 3.07 (s, 1H, CH alkyne), 3.43-3.72 (m, 2H, H-5'), 3.79 (s, 3H, OCH₃), 4.00-4.12 (m, 1H, H-4'), 4.38-4.50 (m, 1H, H-3'), 6.15 (t, *J =* 6.1 Hz, 1H, H-1'), 6.80-7.55 (m, 14H, trityl), 8.15 (s, 1H, H-6); ¹³C NMR (CDCl₃) *δ*: 41.1 (C-2'), 55.4 (OCH₃), 61.9 (C-5'), 70.9 (C-3'), 75.0 (C), 81.7 (CH alkyne), 83.4 (C-1'), 85.9 (C-4'), 87.5 (C), 98.5 (C), 113.6 (CH), 127.4 (CH), 128.2 (CH), 130.4 (CH), 134.9 (C), 143.8 (C), 143.8 (C), 145.3 (C-6), 149.5 (C=O), 159.0 (C), 161.8 (C).
Step b): 5-Ethynyl-1-(4-fluoro-5-trityloxymethyltetrahydrofuran-2-yl)-1*H*-pyrimidine-2,4-dione (**20**)
   To a solution of the product of Step a) (1.26 g, 2.4 mmol) in dry dichloromethane (77.0 mL) at 0°C is added dropwise diethylaminosulfur trifluoride (0.6mL, 4.8mmol), and the reaction mixture is stirred for 15 minutes. Then a saturated NaHCO₃ solution (50.0 mL) is added, the aqueous layer is extracted with CH₂Cl₂ (50 mL x3), the organic layers are washed with water (50 mL), dried over MgSO₄, filtered through a fritted glass funnel and concentrated under reduced pressure to afford the crude product, which is submitted to a flash silica gel column chromatography (eluent: hexanes/EtOAc: 5/5) to give 20 (805.0 mg, 64 %) as a pale yellow solid. [*α*]_{D}²⁰ +12.0 (c 1.0, CHCl₃); mp 54 °C; ¹H NMR (CDCl₃) *δ*: 2.12-2.46 (m, 1H, H-2'a), 2.68-2.90 (m, 1H, H-2'b), 2.88 (s, 1H, CH alkyne), 3.35-3.48 (m, 2H, H-5'), 3.80 (s, 3H, OCH₃), 4.37 (d, *J* = 27.4 Hz, 1H, H-4'), 5.28 (dd, *J* = 4.6 Hz, 53.6 Hz, 1H, H-3'), 6.34 (dd, *J* = 5.3 Hz, 9.0 Hz, 1H, H-1'), 6.80-7.50 (m, 14H, trityl), 8.10 (s, 1H, H-6); ¹³C NMR (CDCl₃) *δ*: 39.7 (d, *J* = 84.2 Hz, C-2'), 55.4 (OCH₃), 63.4 (d, *J* = 40.9 Hz, C-5'), 82.2 (CH alkyne), 84.8 (d, *J* = 101.1 Hz, C-4'), 85.7 (C-1'), 87.8 (C), 94.5 (d, *J* = 710.0 Hz, C-3'), 99.7 (C), 113.6 (CH), 127.4 (CH), 128.3 (CH), 128.4 (CH), 130.5 (CH), 134.6 (C), 143.4 (C-6), 143.6 (C), 143.7 (C), 149.2 (C=O), 159.0 (C x2);
   General procedure for di-halogenation of 5-ethynyl compound
   5-Ethynyl nucleoside of example 37, Step b) (0.5 mmol) is dissolved in dry acetonitrile (5.0 mL) and the reaction mixture is cooled to an ice bath. A solution of the halogenating reagent (i.e. I₂, Ibr or ICI; 0.6 mmol) in dry CH₃CN (1.0 mL) is added dropwise at 0°C and the reaction mixture is stirred until completion (typically 15 min.-2 hours, checked by TLC). The reaction mixture is quenched at 0°C by a saturated Na₂SO₃ solution (2 mL) then extracted with EtOAc (10 mL x 3). The organic layer is washed with water (5 mL x2) and brine (10 mL), then dried over MgSO₄, filtered through a fritted glass funnel and concentrated under reduced pressure to afford the crude product, which is submitted to a flash silica gel column chromatography (eluent: hexanes/EtOAc: 8/2 to 3/7) to give the desired di-halogenated compound.
Step c): 5-(1,2-Diiodovinyl)-1-(4-fluoro-5-trityloxymethyltetrahydrofuran-2-yl)-1*H-*pyrimidine-2,4-dione **(24a)**
   Prepared from compound **20** of step b) using I₂ with the typical procedure described before to give **24a** (32 %) as an orange solid. [*α*]_{D}²⁰ +4.5 (c 1.0, CHCl₃); mp 58 °C; ¹H NMR (CDCl₃) *δ*: 2.05-2.35 (m, 1H, H-2'a), 2.67-2.86 (m, 1H, H-2'b), 3.43 (d, *J* = 3.8 Hz, 2H, H-5'), 3.81 (s, 3H, OCH₃), 4.34 (td, *J* = 3.5 Hz, 27.0 Hz, 1H, H-4'), 5.23 (dd, *J* = 4.7 Hz, 53.7 Hz, 1H, H-3'), 6.33 (dd, J = 5.0 Hz, 9.1 Hz, 1 H, H-1'), 6.75-7.50 (m, 15H, CH alkene, trityl), 7.63 (s, 1H, H-6), 9.00 (se, 1H, NH); ¹³C NMR (CDCl₃) δ: 39.4 (d, *J* = 84.2 Hz, C-2'), 55.5 (OCH₃), 63.1 (d, *J* = 38.5 Hz, C-5'), 84.5 (d, *J* = 101.0 Hz, C-4'), 85.3 (C), 85.5 (CH alkene), 87.5 (C), 87.7 (C-1'), 94.1 (d, *J* = 710.0 Hz, C-3'), 113.6 (CH), 118.1 (C), 127.4 (CH), 128.3 (CH), 128.4 (CH), 130.5 (CH), 134.8 (C), 139.2 (C-6), 143.7 (C), 143.8 (C), 149.5 (C=O), 158.9 (C), 159.0 (C).
Step d): 5-(1,2-Diiodovinyl)-1-(4-fluoro-5-hydroxymethyltetrahydrofuran-2-yl)-1*H-*pyrimidine-2,4-dione **(25a)**
   Prepared from compound **24a**, using the typical procedure described before to give **25a** (51%) as a yellow solid. [*α*]_{D}²⁰ +16.0 (c 1.0, CH₃OH); mp 112 °C; UV (MeOH) λₘₐₓ 232 nm, 274 nm; ¹H NMR (CD₃OD) *δ*: 2.13-2.47 (m, 1H, H-2'a), 2.50-2.77 (m, 1H, H-2'b), 3.80 (d, *J* = 2.8 Hz, 2H, H-5'), 4.32 (td, *J* = 2.9 Hz, 27.0 Hz, 1H, H-4'), 5.30 (dd, *J* = 4.6 Hz, 53.7 Hz, 1H, H-3'), 6.36 (dd, *J* = 5.6 Hz, 9.0 Hz, 1H, H-1'), 7.53 (s, 1H, CH alkene), 8.28 (s, 1H, H-6); ¹³C NMR (CD₃OD) *δ*: 40.3 (d, *J* = 81.9 Hz, C-2'), 62.7 (d, *J* = 43.3 Hz, C-5'), 87.0 (C-1'), 87.3 (d, *J* = 96.3 Hz, C-4'), 87.3 (C), 88.1 (CH alkene), 96.0 (d, *J* = 698.0 Hz, C-3'), 119.2 (C), 142.2 (C-6), 151.5 (C=O), 161.8 (C=O).

Compounds of examples 38 and 39 are obtained in a similar way starting from compounds **24b** and **24c** respectively.

### Example 38: 5-(1-Bromo-2-iodovinyl)-1-(4-fluoro-5-hydroxymethyltetrahydrofuran-2-yl)-1H-pyrimidine-2,4-dione (25b).

Prepared from compound 24b, using the typical procedure described before to give the title compound (52 %) as a pale yellow solid. [*α*]_{D}²⁰ +13.0 (c 0.6, CH₃OH); mp 107 °C; UV (MeOH) λₘₐₓ 296 nm; ¹H NMR (CD₃OD) δ: 2.14-2.47 (m, 1H, H-2'a), 2.52-2.78 (m, 1H, H-2'b), 3.80 (d, *J* = 3.0 Hz, 2H, H-5'), 4.31 (td, *J* = 2.9 Hz, 27.0 Hz, 1H, H-4'), 5.30 (dd, *J* = 4.7 Hz, 53.7 Hz, 1H, H-3'), 6.36 (dd, *J =* 5.6 Hz, 9.0 Hz, 1H, H-1'), 7.30 (s, 1H, CH alkene), 8.36 (s, 1H, H-6); ¹³C NMR (CD₃OD) *δ*: 40.3 (d,C-2'), 62.7 (d, C-5'), 83.2 (CH alkene), 87.0 (C-1'), 87.3 (d, C-4'), 87.3 (C), 96.0 (d, C-3'), 115.5 (C), 116.0 (C), 143.9 (C-6), 151.5 (C=O), 161.8 (C=O).

### Example 39: 5-(1-Chloro-2-iodovinyl)-1-(4-fluoro-5-hydroxymethyltetrahydrofuran-2-yl)-1 H-pyrimidine-2,4-dione (25c)

Prepared from compound **24c**, using the typical procedure described before to give the title compound (76 %) as an orange solid. [*α*]_{D}²⁰ +17.0 (c 0.7, CH₃OH); mp 87 °C; UV (MeOH) λₘₐₓ 274 nm; ¹H NMR (CD₃OD) *δ*: 2.13-2.48 (m, 1H, H-2'a), 2.52-2.75 (m, 1H, H-2'b), 3.80 (d, *J* = 2.8 Hz, 2H, H-5'), 4.30 (td, *J* = 2.8 Hz, 26.8 Hz, 1H, H-4'), 5.30 (dd, *J* = 4.6 Hz, 53.5 Hz, 1H, H-3'), 6.37 (dd, *J* = 5.7 Hz, 8.8 Hz, 1H, H-1'), 7.06 (s, 1H, CH alkene), 8.38 (s, 1H, H-6); ¹³C NMR (CD₃OD) δ: 40.2 (d, *J* = 84.2 Hz, C-2'), 62.7 (d, *J* = 43.3 Hz, C-5'), 81.1 (CH alkene), 87.0 (C-1'), 87.4 (d, *J* = 93.9 Hz, C-4'), 96.2 (d, *J* = 698.0 Hz, C-3'), 114.5 (C), 128.9 (C), 146.0 (C-6), 151.2 (C=O), 164.2 (C=O);

### Example 40: 1-(4-Fluoro-5-hydroxymethyltetrahydrofuran-2-yl)-5-iodoethynyl-1H-pyrimidine-2,4-dione (22)

Step a): 1-(4-Fluoro-5-trityloxymethyl-tetrahydro-furan-2-yl)-5-iodoethynyl-1*H*-pyrimidine-2,4-dione (**21**)
   5-Ethynyl nucleoside **20** (0.5 mmol) was dissolved in dry acetonitrile (5.0 mL) and the reaction mixture is cooled to an ice bath. A solution of the halogenating reagent (i.e. IDCP; 0.7 mmol) and Ag(coll)₂ClO₄ (11.0mg) are added and the reaction mixture is stirred in the dark at rt (typically 2 h-20 h, checked by TLC). The reaction mixture is quenched at 0°C by a saturated Na₂SO₃ solution (2 mL) then extracted with EtOAc (10 mL x4). The organic layer is washed with water (10 mL), a 1 M HCl solution (5 mL x3), water (5 mL x2), and brine (10 mL), then dried over MgSO₄, filtered through a fritted glass funnel and concentrated under reduced pressure to afford the crude product, which is submitted to a flash silica gel column chromatography (eluent: hexanes/EtOAc: 8/2 to 3/7) to give **21** (26 %) as yellow solid. [*α*]_{D}²⁰ +122.0 (c 1.0, CHCl₃); mp 73 °C; ¹H NMR (CDCl₃) *δ*: 2.15-2.48 (m, 1H, H-2'a), 2.68-2.93 (m, 1H, H-2'b), 3.30-3.56 (m, 2H, H-5'), 3.81 (s, 3H, OCH₃), 4.38 (d, *J* = 27.0 Hz, 1H, H-4'), 5.32 (dd, *J* = 4.4 Hz, 53.4 Hz, 1H, H-3'), 6.33 (dd, *J* = 5.3 Hz, 9.1 Hz, 1H, H-1'), 6.75-7.50 (m, 14H, trityl), 8.01 (s, 1H, H-6), 9.20 (se, 1H, NH); ¹³C NMR (CDCl₃) *δ*: 12.1 (CCI), 39.8 (d, *J* = 86.6 Hz, C-2'), 55.4 (OCH₃), 63.4 (d, *J* = 40.9 Hz, C-5'), 84.2 (C), 84.9 (d, *J* = 101.1 Hz, C-4'), 86.0 (C-1'), 87.8 (C), 94.5 (d, *J* = 707.6 Hz, C-3'), 101.0 (C), 113.6 (CH), 127.5 (CH), 127.9 (C), 128.1 (CH), 128.2 (CH), 130.4 (CH), 134.6 (C), 143.5 (C), 143.7 (C-6), 143.8 (C), 149.2 (C=O), 159.0 (C), 161.6 (C).
Step b): 1-(4-Fluoro-5-hydroxymethyltetrahydrofuran-2-yl)-5-iodoethynyl-1*H*-pyrimidine-2,4-dione (**22**)
   A solution of the protected nucleoside **21** (0.3 mmol) in acetic acid 80 % (30 mL) is stirred until completion (checked by TLC, typically 18 hours). After evaporation of the solvent, the crude product was submitted to a flash silica gel column chromatography (eluent: hexanes/EtOAc: 2/8) to give **22** (47 %) as a yellow solid. [*α*]_{D}²⁰ -1.5 (c 1.0, CH₃OH); mp 140°C; UV (MeOH) λₘₐₓ 233 nm, 298 nm; ¹H NMR (CD₃OD) δ: 2.11-2.45 (m, 1H, H-2'a), 2.46-2.75 (m, 1H, H-2'b), 3.79 (d, *J* = 3.2 Hz, 2H, H-5'), 4.29 (td, *J* = 2.9 Hz, 27.0 Hz, 1H, H-4'), 5.27 (dd, *J* = 4.9 Hz, 53.9 Hz, 1H, H-3'), 6.28 (dd, *J* = 5.5 Hz, 8.9 Hz, 1H, H-1'), 8.30 (s, 1H, H-6); ¹³C NMR (CD₃OD) *δ*: 15.8 (CCI), 39.8 (d, *J* = 81.8 Hz, C-2'), 62.5 (d, *J* = 43.3 Hz, C-5'), 85.2 (C), 87.0 (C-1'), 87.3 (d, *J* = 96.3 Hz, C-4'), 96.0 (d, *J* = 700.4 Hz, C-3'), 101.5 (C), 146.0 (C-6), 151.1 (C=O), 164.4 (C=O).

### Example 41: 1-(4-Azido-5-hydroxymethyltetrahydrofuran-2-yl)-5-(1,2-diiodovinyl)-1H-pyrimidine-2,4-dione (31 a)

Step a): 1-(4-Azido-5-trityloxymethyltetrahydrofuran-2-yl)-5-ethynyl-1*H*-pyrimidine-2,4-dione **(26)**
   A solution of **18** (100 mg, 0.16 mmol) and NaN₃ (35 mg, 0.54 mmol) in DMF (5 mL) is refluxed overnight. After evaporation of volatiles, the crude product is submitted to a flash silica gel column chromatography (eluent: hexanes/EtOAc: 2/8) to give 26 (61.0 mg, 70%) as a pale yellow solid. [*α*]_{D}²⁰ +13 (c 0.8, CH₃OH); mp 110°C; ¹H NMR (CDCl₃) *δ*: 2.35-2.46 (m, 1H, H-2'a), 2.52-2.62 (m, 1H, H-2'b), 2.89 (s, 1H, CH alkyne), 3.37-3.52 (m, 2H, H-5'), 3.82 (s, 3H, OCH₃), 4.00-4.04 (m, 1H, H-4'), 4.28-4.34 (m, 1H, H-3'), 6.15 (t, *J* = 6.1 Hz, 1H, H-1'), 6.88 (d, *J* = 8.9 Hz, 2H, trityl), 7.21-7.51 (m, 14H, trityl), 8.08 (s, 1H, H-6); ¹³C NMR (CDCl₃) *δ*: 38.6 (C-2'), 55.3 (OCH₃), 60.4 (C-3'), 62.7 (C-5'), 82.3 (CH alkyne), 84.0 (C-4'), 85.5 (C-1'), 87.6 (C), 99.4 (C), 113.6 (CH x2), 127 .3 (CH), 128.1 (CH), 128.2 (CH), 130.4 (CH), 134.7 (C), 143.7 (C), 149.2 (C), 158.9 (C), 161.6 (C);
Step b): 1-(4-Azido-5-trityloxymethyl-tetrahydro-furan-2-yl)-5-(1,2-diiodovinyl)-1*H-*pyrimidine-2,4-dione (**30a**)
   Prepared from compound of Step a), using I₂ with the typical procedure described before for the preparation of **24a** to give **30a** (52 %) as a pale yellow solid. [*α*]_{D}²⁰ +16 (c 1.0, CH₃OH); mp 139°C; ¹H NMR (CDCl₃) *δ*: 2.23-2.34 (m, 1H, H-2'a), 2.51-2.61 (m, 1H, H-2'b); 3.36-3.49 (m, 2H, H-5'), 3.81 (s, 3H, OCH₃), 3.98-4.05 (m, 1H, H-4'), 4.16-4.26 (m, 1H, H-3'), 6.16 (t, *J* = 6.6 Hz, 1H, H-1'), 6.87 (d, *J =* 8.8 Hz, 2H, trityl), 7.21-7.43 (m, 13H, trityl, CH alkene), 7.58 (s, 1H, H-6); ¹³C NMR (CDCl₃) *δ*: 38.1 (C-2'), 55.2 (OCH₃), 60.4 (C-3'), 62.8 (C-5'), 83.5 (C-4'), 85.1 (C-1'), 87.1 (C), 87.2 (CH alkene), 113.3 (CH), 117.6 (C), 127.1 (CH), 128.0 (CH), 128.1 (CH), 130.2 (CH), 135.6 (C), 139.0 (C-6), 143.5 (C), 143.6 (C), 149.1 (C=O), 158.6 (C), 158.7 (C).
Step c): 1-(4-Azido-5-hydroxymethyltetrahydrofuran-2-yl)-5-(1,2-diiodovinyl)-1*H-*pyrimidine-2,4-dione(**31a**)
   Prepared from compound **30a** using the typical procedure described before for the preparation of **24a** to give **31a** (78%) as a pale yellow gum. [*α*]_{D}²⁰ +17 (c 1.0, CH₃OH); UV (MeOH) λₘₐₓ 225 nm, 284 nm; ¹H NMR (DMSO-*d*_{*6*}) *δ*: 2.34-2.43 (m, 2H, H-2'), 3.56-3.71 (m, 2H, H-5'), 3.85-3.90 (m, 1H, H-4'), 4.35-4.41 (m, 1H, H-3'), 5.26 (t, *J* = 4.9 Hz, 1H, OH), 6.09 (t, *J* = 6.3 Hz, 1H, H-1'), 7.61 (s, 1H, CH alkene), 8.06 (s, 1H, H-6'), 11.68 (s, 1H, NH); ¹³C NMR (DMSO-*d*_{*6*}) *δ*: 37.5 (C-2'), 60.4 (C-3'), 61.4 (C-5'), 85.0 (C-1', C-4'), 88.9 (C-I), 90.2 (CH alkene), 117.7 (C-5), 140.5 (C-6), 150.1 (C=O), 159.9 (C=O).

Compounds of examples 42 and 43 are obtained in a similar way, starting from compounds 30b and 30c respectively.

### Example 42: 1-(4-Azido-5-hydroxymethyltetrahydrofuran-2-yl)-5-(1-bromo-2-iodo-vinyl)-1H-pyrimidine-2,4-dione (31 b)

Prepared from compound **30b** using the typical procedure described before to give **31b** (53%) as a pale yellow gum. [*α*]_{D}²⁰ +11 (c 1.0, CH₃OH); UV (MeOH) λₘₐₓ 229 nm, 276 nm; ¹H NMR (DMSO-*d*_{*6*}) *δ*: 2.36-2.43 (m, 2H, H-2'), 3.57-3.69 (m, 2H, H-5'); 3.86-3.91 (m, 1H, H-4'), 4.35-4.42 (m, H-3'), 5.28 (brs, 1H, OH), 6.09 (t, *J* = 5.9 Hz, 1H, H-1'), 7.45 (s, 1H, CH alkene), 8.18 (s, 1H, H-6'), 11.7 (s, 1H, NH); ¹³C NMR (DMSO-*d*_{*6*}) *δ*: 37.0 (C-2'), 60.0 (C-3'), 60.7 (C-5'), 84.6 (C-1', C-4'), 85.6 (CH alkene), 113.9 (C), 114.2 (C), 141.9 (C-6), 149.6 (C=O), 159.4 (C=O);

### Example 43: 1-(4-Azido-5-hydroxymethyltetrahydrofuran-2-yl)-5-(1-chloro-2-iodo-vinyl)-1H-pyrimidine-2,4-dione (31c)

Prepared from compound **30c** using the typical procedure described before to give **31c** (57%) as a pale yellow gum. [*α*]_{D}²⁰ +28 (c 1.0, CH₃OH); UV (MeOH) λₘₐₓ 214 nm, 273 nm; ¹H NMR (DMSO-*d*_{*6*}) *δ*: 2.33-2.44 (m, 2H, H-2'), 3.33-3.71 (m, 1H, H-5'), 3.86-3.91 (m, 1H, H-4'), 4.35-4.42 (m, 1H, H-3'), 5.28 (t, *J* = 4.9 Hz, 1H, OH), 6.09 (t, *J* = 6.2 Hz, 1H, H-1'), 7.23 (s, 1H, CH alkene), 8.22 (s, 1H, H-6), 11.73 (s, 1H, NH); ¹³C NMR (DMSO-*d*_{*6*}) *δ*: 37.7 (C-2'), 60.7 (C-3'), 61.3 (C-5'), 84.5 (CH), 85.2 (CH x2), 113.0 (C), 127.4 (C), 143.1 (C-6), 150.3 (C=O), 160.1 (C=O).

### Example 44: 1-(4-Azido-5-hydroxymethyl-tetrahydro-furan-2-yl)-5-iodoethynyl-1H-pyrimidine-2,4-dione (28)

Prepared from compound **26** using a similar procedure as the one disclosed above for Example 40, to give the title compound 60 % as a pole yellow gum, [*α*]_{D}²⁰ +15 (c 1.0, CH₃OH); UV (MeOH) λₘₐₓ 236 nm, 295 nm; ¹H NMR (DMSO-*d*_{*6*}) *δ*: 2.24-2.34 (m, 1H, H-2'a), 2.39-2.46 (m, 1H, H-2'b), 3.54-3.72 (m, 2H, H-5'), 3.79-3.84 (m, 1H, H-4'), 4.34-4.41 (m, 1H, H-3'), 5.30 (t, *J* = 5.3 Hz, 1H, OH), 6.01 (t, *J* = 6.0 Hz, 1H, H-1'), 8.22 (s, 1H, H-6), 11.66 (s, 1H, NH); ¹³C NMR (DMSO-*d*_{*6*}) *δ*: 19.8 (C), 36.8 (C-2'), 59.2 (C-3'), 60.2 (C-5'), 84.3 (C-1', C-4'), 84.8 (C), 99.0 (C), 144.6 (C-6), 149.3 (C=O), 161.7 (C=O).

### Biological evaluation

**Antiviral and Cytotoxicity assays for HCV.** Huh7 cells harbouring the subgenomic HCV replicon BM4-5 (J.T. Guo et al., J. Virol. 2001, 75: 8516-8523) were used in this study. Cells were maintained in Dulbecco's modified Eagle's medium high glucose 4.5 g/I (LifeTechnologies) supplemented with 10% fetal bovine serum, 1% L-glutamine, 1% penicillin and streptomycin, 1% L-Pyruvate and 500 µg/ml of geneticin (G418, Invitrogen). Geneticin was used to select cells permitting the HCV RNA replication. Cells were passaged every four days with a 1:4 ratio.

Cells were seeded in 6-well plates at a density of 2.5 x 10⁵ cells per well, sixteen hours before the beginning of treatment. Cells were treated with the molecules administered at different concentrations in complete medium that did not contain geneticin. The administration of each drug was renewed every day for three consecutive days. Ribavirin (ICN Pharmaceuticals), mycophenolic acid (MPA, Sigma), and interferon alpha-2b (IntronA® ) were used in the same conditions as positive controls. The concentrations used for these drugs were respectively: 0; 0.5; 1; 2; 4; 8; 16; 32; 64; 128; 256, and 512 µM for ribavirin, 0; 2.5; 5; 10; 20; 40 µM for mycophenolic acid, and 0; 0.1; 1; 10; 100 and 1000 UI /mL for IFN alpha-2b. Total RNA was extracted at the end of treatment (24 hours after the last day of treatment) with the "Extract all" reagent (Eurobio), which is a mix of phenol and guanidinium thiocyanate. Northern blot analysis was then performed using the NorthernMax™-Gly kit (Ambion), following manufacturer's instructions. Five micrograms of total RNA were denaturated in glyoxal buffer at 50°C for 30 minutes, separated by 1.1% agarose gel electrophoresis, and then transferred for 12 hours onto a charged nylon membrane (HybondN+, Amersham). Hybridization was carried out with three different [³²P]CTP-labelled riboprobes obtained by *in vitro* transcription (Riboprobe *in vitro* transcription system, Promega). Two probes were complementary to the NS5A region of the HCV genome of negative polarity and positive polarity. A third probe was complementary to the beta-actin mRNA and obtained by *in vitro* transcription from a specific plasmid (pTRI beta actin human, reference 7424, Ambion). First, the blot was hybridized with the riboprobes directed against the negative strand of HCV RNA and beta-actin mRNA, respectively. After one night of hybridization at 68°C, the membrane was washed, then exposed to X-ray film and a phosphor screen (phosphorimager). This screen was then scanned and quantitative analysis was achieved using ImageQuant software. The amount of beta-actin mRNA was used as an internal loading control to standardize the amount of HCV RNA detected. The same membrane was subsequently hybridized with the negative sense riboprobe to determine the level of positive strand HCV RNA, using the same approach.

For cell viability assays, cells were seeded in 96-well plates at a density of 12,500 cells per well. They were treated by the different molecules with the same concentrations and conditions than those used for the antiviral assays. Then, cell viability was measured by neutral red assay. Neutral red specifically colors lysosomes and its accumulation depends on cellular membrane integrity. The yield of neutral red incorporated in cells is proportional to the number of living cells. At the end of treatment, culture medium was removed; cells were washed by PBS, then coloured with neutral red at 0.005% for 3 hours at 37°C. Cells were then fixed 1 minute by formol calcium and lysed by atreatment with a vol/vol mixture of acetic acid and ethanol. After 15 minutes incubation, absorbance was read at 490nm.

**Antiviral and cytotoxicity assays for BVDV.** Noncytopathic-BVDV-free MDBK cells (European Collection of Animal Cell Cultures, Porton Down, U.K.) were kindly provided by Dr N. Zitzmann (Oxford University). Cells were propagated in DMEM F12 (Eurobio) supplemented with 10% horse serum (Life Technologies), 1% L-glutamin (Gibco), 1% penicillin and streptomycin (Gibco). The NADL cytopathic (CP) BVDV strain was obtained from ATCC: VR-504.
MDBK cells were seeded in microwell plates (96 wells) at a density of 1.10⁵ cells per well, then infected with BVDV (strain NADL) (at a dilution inducing 100% cytopathic effect three days post inoculation, i.e. approximately 10 plaque forming units (pfu) per well at three days post infection) for one hour, at 37°C. After a wash with DMEM, infected cells were incubated for three days in the presence or absence of drug; each concentration of drug was added in 12 consecutive wells. The appearance of CPE was visually checked the third day post inoculation to evaluate the IC50 on BVDV (strain NADL), according to the different conditions of treatment. By definition, the IC50 is the concentration of drug that inhibits 50% of the CPE in comparison to cells inoculated but untreated presenting 100% of CPE.

Uninfected MDBK cells were grown in the absence or presence of varying concentrations of drug tested. After 3 days, toxicity was evaluated by neutral red coloration as described above.

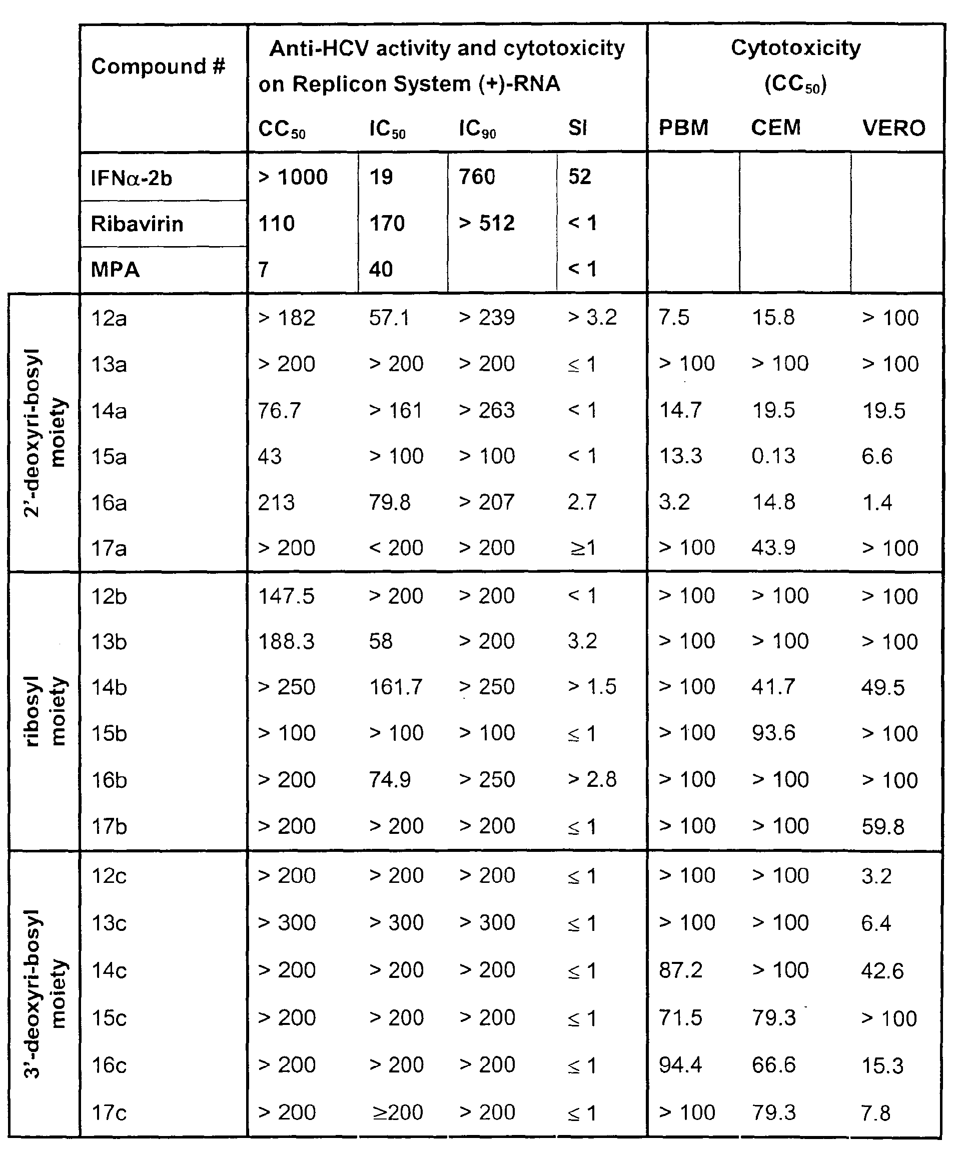

**Table 2: Neutral red testing and antiviral assay on BVDV strain NADL expressed in µM**

| **Compound #** | **CC**_{**50**} **(Neutral Red)** | **EC**_{**50**} **ECP inhibition (3**^{**rd**} **day p.i.)** | **SI** |
|---|---|---|---|
| Ribavirin | > 100 | 36 µM | > 2.77 |
| 12a-c, 13b,14a-c, 15c, 16a-c | > 100 | > 100 | < 1 |

It should be clearly understood that the invention defined by the attached claims is not limited to the particular embodiments indicated in the above description, but encompasses the variants which depart neither from the context nor from the spirit of the present invention.

## Claims

1. Use of an uridine derivative of formula (I): 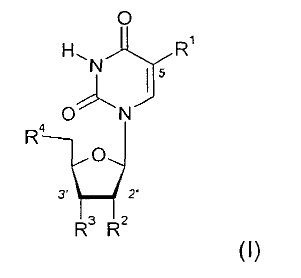 wherein
- R¹ represents monohalogenated alkynyl or dihalogenated alkenyl;
- R² is chosen from among hydrogen, hydroxyl, -O-alkyl, -O-CO-alkyl and halogen;
- R³ is chosen from among hydrogen, hydroxyl, -O-alkyl, -O-CO-alkyl, halogen, -SH, -S-alkyl and N³; and
- R⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms,
for the preparation of a drug having antiviral activity against a Flaviviridae.

2. The use according to claim 1, wherein the uridine derivative of formula (I) presents the following characteristics:
- R¹ represents -C ≡C-X or -C(Y)=CH(X);
- X and Y each independently represents halogen;
- R² is chosen from among hydrogen, hydroxyl, -O-alkyl, -O-CO-alkyl and halogen;
- R³ is chosen from among hydrogen, hydroxyl, -O-alkyl, -O-CO-alkyl, halogen, -SH, -S-alkyl and N³; and
- R⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms.

3. The use according to claim 2, wherein R¹ represents chloroethynyl, bromoethynyl, iodoethynyl or -C(Y)=CH(X), where Y is chosen from among chlorine, bromine and iodine, and X is bromine or iodine.

4. The use according to claim 2, wherein
- R¹ represents chloroethynyl, bromoethynyl, iodoethynyl or -C(Y)=CH(X), where Y is chosen from among chlorine, bromine and iodine, and X is bromine or iodine;
- R² is chosen from among hydrogen, hydroxyl, and -O-alkyl;
- R³ is chosen from among hydrogen, hydroxyl, -O-alkyl, -SH, and -S-alkyl;
- R² and R³ not simultaneously being each hydrogen; and
- R⁴ is chosen from among hydroxyl, -O-alkyl, -O-CO-alkyl, -O-phosphate, -O-diphosphate, -O-triphosphate and -O-phosphonate
as well as their possible tautomers, their possible pharmaceutically acceptable addition salts with an acid or a base, and their N-oxide forms.

5. The use according to claim 1, wherein the uridine derivative is chosen from among the following compounds:
• (*E*)-5-(1-Chloro-2-iodovinyl)-2'-deoxyuridine,
• (*E*)-5-(1,2-Diiodovinyl)-2'-deoxyuridine,
• 5-(2-Iodoethynyl)-2'-deoxyuridine,
• (*E*)-5-(1-Bromo-2-iodovinyl)-2'-deoxyuridine,
• (*E*)-5-(1,2-Dibromovinyl)-2'-deoxyuridine,
• 5-(2-Bromoethynyl)-2'-deoxyuridine,
• (*E*)-5-(1-Chloro-2-iodovinyl)-uridine,
• (*E*)-5-(1,2-Diiodovinyl)-uridine,
• 5-(2-Iodoethynyl)-uridine,
• (*E*)-5-(1-Bromo-2-iodovinyl)-uridine,
• (*E*)-5-(1,2-Dibromovinyl)-uridine, and
• 5-(2-bromoethynyl)-uridine,
as well as its possible tautomers, its possible pharmaceutically acceptable addition salts with an acid or a base, and its N-oxide forms.

6. The use according to any one of claims 1 to 5, wherein an uridine derivative of formula (I) as well as its possible tautomers, its possible pharmaceutically acceptable addition salts with an acid or a base, and its N-oxide forms, and an interferon are used for the preparation of a drug having antiviral activity against a Flaviviridae.

7. The use according to any one of claims 1 to 6, for the preparation of a drug having antiviral activity against hepatitis C virus (HCV).

8. A drug having an antiviral activity against a Flaviviridae, comprising a compound of formula (I'): 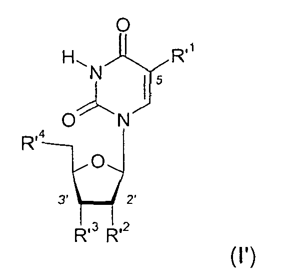 wherein
- R'¹ represents dihalogenated alkenyl;
- R'², R'³ and R'⁴ have the same definitions as R², R³ and R⁴, respectively, in the above formula (I);
as well as its possible tautomers, its possible pharmaceutically acceptable addition salts with an acid or a base, and its N-oxide forms.

9. The drug according to claim 8, wherein the compound of formula (I') presents the following characteristics:
- R'¹ represents -C(Y)=CH(X);
- X and Y each independently represents halogen;
- R'² is chosen from among hydrogen, hydroxyl, -O-alkyl, -O-CO-alkyl and halogen;
- R'³ is chosen from among hydrogen, hydroxyl, -O-alkyl, -O-CO-alkyl, halogen, -SH, -S-alkyl and N³; and
- R'⁴ is chosen from among hydroxyl, -O-alkyl, -O-CO-alkyl, -O-phosphate, -O-diphosphate, -O-triphosphate and -O-phosphonate
as well as their possible tautomers, their possible pharmaceutically acceptable addition salts with an acid or a base, and their N-oxide forms.

10. The drug according to claim 9, wherein R'¹ represents -C(Y)=CH(X), where Y is chosen from among chlorine, bromine and iodine, and X is bromine or iodine.

11. The drug according to claim 9, wherein
- R'¹ represents -C(Y)=CH(X), where Y is chosen from among chlorine, bromine and iodine, and X is bromine or iodine.
- R'² is chosen from among hydrogen, hydroxyl, and -O-alkyl;
- R'³ is chosen from among hydrogen, hydroxyl, -O-alkyl, -SH, and -S-alkyl; and
- R'² and R'³ not simultaneously being each hydrogen; and
- R'⁴ is chosen from among hydroxyl, -O-alkyl, -O-CO-alkyl, -O-phosphate, -O-diphosphate, -O-triphosphate and -O-phosphonate,
as well as their possible tautomers, their possible pharmaceutically acceptable addition salts with an acid or a base, and their N-oxide forms.

12. A drug formulation having an antiviral activity against a Flaviviridae comprising
(1) an uridine derivative of formula (I): 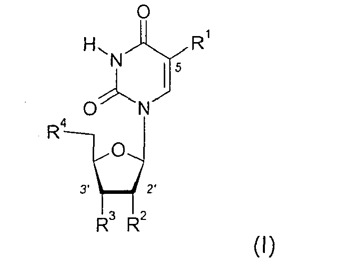 wherein
- R¹ represents monohalogenated alkynyl or dihalogenated alkenyl;
- R² is chosen from among hydrogen, hydroxyl, -O-alkyl, -O-CO-alkyl and halogen;
- R³ is chosen from among hydrogen, hydroxyl, -O-alkyl, -O-CO-alkyl, halogen, -SH, -S-alkyl and N³; and
- R⁴ is chosen from among hydroxyl, -O-alkyl, -O-CO-alkyl, -O-phosphate, -O-diphosphate, -O-triphosphate and -O-phosphonate,
as well as its possible tautomers, its possible pharmaceutically acceptable addition salts with an acid or a base, and its N-oxide forms,
(2) an immunomodulator or another active ingredient having an antiviral activity against a Flaviviridae, and
(3) a pharmaceutically acceptable vehicle, carrier or excipient.

13. The drug formulation according to claim 12, wherein ingredient (2) comprises an interferon.
